(19) Europäisches Patentamt
European Patent Office
Office européen des brevets

(11) EP 4 180 415 B1

(12) EUROPEAN PATENT SPECIFICATION

(45) Date of publication and mention of the grant of the patent:
**26.11.2025 Bulletin 2025/48**

(21) Application number: **21836786.0**

(22) Date of filing: **06.07.2021**

(51) International Patent Classification (IPC):
***C07C 209/68** (2006.01)* ***C07C 211/36** (2006.01)*
***C07C 265/14** (2006.01)*

(52) Cooperative Patent Classification (CPC):
(C-Sets available)
**C07C 209/68; C07C 211/36; C07C 265/14;**
C07C 2601/14 (Cont.)

(86) International application number:
**PCT/JP2021/025394**

(87) International publication number:
**WO 2022/009860 (13.01.2022 Gazette 2022/02)**

# (54) ALIPHATIC DIAMINE ISOMERIZATION METHOD, DIISOCYANATE PRODUCTION METHOD, POLYURETHANE PRODUCTION METHOD, AND POLYURETHANE

VERFAHREN ZUR ISOMERISIERUNG EINES ALIPHATISCHEN DIAMINS, VERFAHREN ZUR HERSTELLUNG VON DIISOCYANAT, VERFAHREN ZUR HERSTELLUNG VON POLYURETHAN UND POLYURETHAN

PROCÉDÉ D'ISOMÉRISATION DE DIAMINE ALIPHATIQUE, PROCÉDÉ DE PRODUCTION DE DIISOCYANATE, PROCÉDÉ DE PRODUCTION DE POLYURÉTHANE ET POLYURÉTHANE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.07.2020 JP 2020117702**

(43) Date of publication of application:
**17.05.2023 Bulletin 2023/20**

(73) Proprietor: **MITSUBISHI GAS CHEMICAL COMPANY, INC.**
**Chiyoda-ku**
**Tokyo 100-8324 (JP)**

(72) Inventors:
- **UENO, Masayoshi**
**Niigata-shi, Niigata 950-3112 (JP)**
- **IIDA, Akifumi**
**Niigata-shi, Niigata 950-3112 (JP)**
- **NAGAMATSU, Kentaro**
**Niigata-shi, Niigata 950-3112 (JP)**

(74) Representative: **Hoffmann Eitle**
**Patent- und Rechtsanwälte PartmbB**
**Arabellastraße 30**
**81925 München (DE)**

(56) References cited:
**WO-A1-2015/041262 WO-A1-2016/143536**
**WO-A1-2016/143537 WO-A1-2016/143537**
**WO-A1-2016/143538 WO-A1-2019/206694**
**JP-A- 2015 147 940 JP-A- 2015 147 940**
**JP-A- S5 699 447**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

(52) Cooperative Patent Classification (CPC): (Cont.)

C-Sets
**C07C 209/68, C07C 211/36**

## Description

### Technical Field

The present invention relates to a method for isomerizing an aliphatic diamine, a method for producing diisocyanate, a method for producing polyurethane, and polyurethane.

### Background Art

Diaminodicyclohexylmethane and dimethyldiaminodicyclohexylmethane are industrially important compounds used as raw materials for epoxy curing agents, polyurethane, and the like. Diaminodicyclohexylmethane has three types of isomers, a cis-cis isomer, a cis-trans isomer, and a trans-trans isomer, attributed to a cyclohexane ring. Similarly, dimethyldiaminodicyclohexylmethane has three types of isomers, a cis-cis isomer, a cis-trans isomer, and a trans-trans isomer, for two amino groups bonded to a cyclohexane ring.

Cyclohexanediamine is an industrially important compound used as a raw material for epoxy curing agents, polyurethane, and the like. The cyclohexanediamine has two types of isomers, a cis isomer and a trans isomer, attributed to a cyclohexane ring.

1,3,3-Trimethyl-1-(aminomethyl)-5-aminocyclohexane (hereinafter, also referred to as "isophoronediamine" or "IPDA") is an industrially important compound used as a raw material for epoxy curing agents, polyurethane, and the like. Isophoronediamine has two types of isomers, a cis isomer and a trans isomer, attributed to a cyclohexane ring.

It is known that the physical properties of polymers using aliphatic diamines such as diaminodicyclohexylmethane, cyclohexanediamine, and isophoronediamine vary depending on the isomer ratio thereof.

For example, Patent Document 1 discloses that a polyurethane obtained from bis(isocyanatocyclohexyl) methane which is obtained by isocyanating diaminodicyclohexylmethane has higher mechanical strength as a content of a trans-trans structure increases.

In addition, trans-1,4-bisisocyanatocyclohexane obtained from the trans isomer of 1,4-cyclohexanediamine is particularly useful as a polyurethane material, and the cis isomer of 1,4-cyclohexanediamine is liquefied and is particularly useful as a curing agent for epoxy resins.

Further, Patent Document 2 discloses that an epoxy resin using isophoronediamine having a high content of a trans isomer as a curing agent has an extended pot life and a decreased maximum exothermic temperature, whereas an epoxy resin using isophoronediamine having a high content of a cis isomer as a curing agent has an extremely high reaction rate.

For this reason, it is extremely important to control the isomer ratio of aliphatic diamines having a cyclohexane ring, such as diaminodicyclohexylmethane, cyclohexanediamine, and isophoronediamine. As a method for controlling the isomer ratio of an aliphatic diamine, various methods have been proposed.

For example, in Patent Document 3, Patent Document 4, and Patent Document 5, as a method for isomerizing diaminodicyclohexylmethane, cyclohexanediamine, or isophoronediamine, methods of realizing an isomerization reaction in the presence of a specific imine compound and one or more selected from the group consisting of an alkali metal, an alkali metal-containing compound, an alkaline earth metal, and an alkaline earth metal-containing compound have been proposed.

### Citation List

#### Patent Document

Patent Document 1: JP 07-188128 A
Patent Document 2: German Patent Application Publication No. 4211454
Patent Document 3: WO 2016/143536
Patent Document 4: WO 2016/143537
Patent Document 5: WO 2016/143538

### Summary of Invention

#### Technical Problem

However, the methods disclosed in Patent Document 3, Patent Document 4, and Patent Document 5 can easily

realize the isomerization reaction of an aliphatic diamine without undergoing a high pressure reaction or a complicated multi-step process, but there is room for improvement in the yield of the isomerization reaction.

The present invention has been made in view of the above problems, and an object of the present invention is to provide a method for isomerizing an aliphatic diamine, which can easily realize an isomerization reaction of the aliphatic diamine, which is an industrially important compound, in a high yield without undergoing a complicated multi-step process.

Solution to Problem

The present inventors have been diligently studied to solve the above problems. As a result, the present inventors have found that the above problems can be solved by an isomerization method including a predetermined isomerization step and using a specific catalyst in a specific blending amount, and have completed the present invention.

That is, the present invention is as follows.

(1) A method for isomerizing an aliphatic diamine, the method including isomerizing an aliphatic diamine represented by Formula (1) below in the presence of:

an imine compound obtained by dehydration condensation of an aliphatic diamine represented by Formula (1) below and an aldehyde and/or a ketone; and

at least one selected from the group consisting of an alkali metal, an alkali metal-containing compound, an alkaline earth metal, and an alkaline earth metal-containing compound,

wherein a ratio of a total amount of the alkali metal, the alkali metal-containing compound, the alkaline earth metal, and the alkaline earth metal-containing compound used to a total amount of the aliphatic diamine used is 1.0 mol% or greater and 4.0 mol% or less:

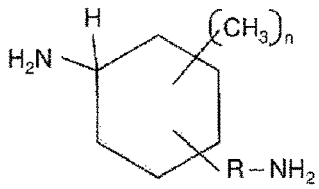

(1)

wherein R represents a single bond or an unsubstituted aliphatic or alicyclic alkylene group having 1 to 8 carbon atoms, and n represents an integer from 0 to 5.

(2) The method for isomerizing the aliphatic diamine according to [1], wherein a ratio of a total amount of the aldehyde and ketone used to the total amount of the aliphatic diamine used is 3.5 mol% or greater and 15.0 mol% or less.

(3) The method for isomerizing the aliphatic diamine according to [1] or [2], wherein the ratio of the total amount of the alkali metal, the alkali metal-containing compound, the alkaline earth metal, and the alkaline earth metal-containing compound used to the total amount of the aliphatic diamine used is 1.0 mol% or greater and 4.0 mol% or less, and the ratio of the total amount of the aldehyde and ketone used to the total amount of the aliphatic diamine used is 3.5 mol% or greater and 10.0 mol% or less.

(4) The method for isomerizing the aliphatic diamine according to any one of [1] to [3], wherein the aliphatic diamine comprises an aliphatic diamine represented by Formula (1A) below:

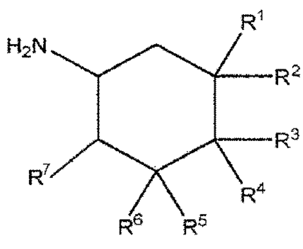

(1 A)

wherein any one of $R^1$, $R^2$, $R^3$, $R^4$, $R^5$, $R^6$, and $R^7$ is an aminoalkyl group represented by $-R-NH_2$, and the others of $R^1$,

$R^2$, $R^3$, $R^4$, $R^5$, $R^6$, and $R^7$ each independently represent a hydrogen atom or a methyl group, and R represents a single bond or an unsubstituted aliphatic or alicyclic alkylene group having 1 to 8 carbon atoms.

(5) The method for isomerizing the aliphatic diamine according to any one of [1] to [4], wherein the isomerization is a step of isomerizing the aliphatic diamine in the presence of the imine compound and at least one selected from the group consisting of sodium amide, metallic sodium, and sodium hydride.

(6) The method for isomerizing the aliphatic diamine according to any one of [1] to [5], wherein the imine compound is an imine compound obtained by dehydration condensation of the aliphatic diamine with an aldehyde having an aromatic ring and/or a ketone having an aromatic ring.

(7) The method for isomerizing the aliphatic diamine according to any one of [1] to [6], wherein the aliphatic diamine is diaminodicyclohexylmethane.

(8) The method for isomerizing the aliphatic diamine according to any one of [1] to [6], wherein the aliphatic diamine is 1,3,3-trimethyl-1-(aminomethyl) aminocyclohexane.

(9) The method for isomerizing the aliphatic diamine according to any one of [1] to [6], wherein the aliphatic diamine is cyclohexanediamine.

(10) The method for isomerizing the aliphatic diamine according to any one of [1] to [6], wherein the aliphatic diamine is dimethyldiaminodicyclohexylmethane.

(11) The method for isomerizing the aliphatic diamine according to any one of [1] to [6], wherein the aliphatic diamine is methylcyclohexanediamine.

(12) The method for isomerizing the aliphatic diamine according to any one of [1] to [6], wherein the aliphatic diamine comprises an aliphatic diamine represented by Formula (1B) below:

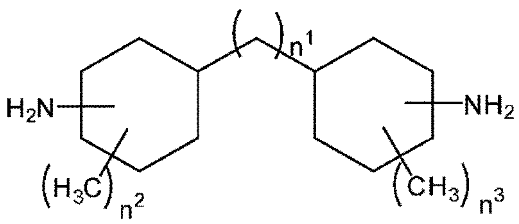

(1 B)

where $n^1$ is an integer from 0 to 2, and $n^2$ is an integer from 0 to 5, $n^3$ is an integer from 0 to 2, and a sum of $n^1$ and $n^3$ is 2 or less).

(13) A method for producing diisocyanate, the method including:

isomerizing an aliphatic diamine by the method described in any one of [1] to [12]; and
obtaining diisocyanate using the isomerized aliphatic diamine as a raw material.

(14) A method for producing polyurethane, the method including:

isomerizing an aliphatic diamine by the method described in any one of [1] to [12];
obtaining diisocyanate using the isomerized aliphatic diamine as a raw material; and
reacting the diisocyanate obtained in the previous step with polyol to obtain polyurethane.

(15) Polyurethane including a structure derived from the diisocyanate produced by the production method according to [13], wherein the production method according to [13] uses isomerized aliphatic diamine as a raw material, which has a trans-trans isomer proportion of 50% or greater.

Advantageous Effects of Invention

According to the present invention, as compared with the known technique, it is possible to provide a method for isomerizing an aliphatic diamine, which can easily realize an isomerization reaction of the aliphatic diamine, which is an industrially important compound, in a high yield without undergoing a complicated multi-step process.

Brief Description of Drawings

FIG. 1 illustrates a change in isomer proportion over time in Example 1.

Description of Embodiments

Hereinafter, embodiments of the present invention (hereinafter, referred to as "the present embodiment") will be described in detail; however, the present invention is not limited to these embodiments, and various modifications may be made without departing from the scope of the invention as defined by the appended claims.

[Method for isomerizing aliphatic diamine]

A method for isomerizing an aliphatic diamine includes isomerizing an aliphatic diamine represented by Formula (1) below in the presence of: an imine compound obtained by dehydration condensation of an aliphatic diamine represented by Formula (1) below and an aldehyde and/or a ketone; and at least one selected from the group consisting of an alkali metal, an alkali metal-containing compound, an alkaline earth metal, and an alkaline earth metal-containing compound, wherein a ratio of a total amount of the alkali metal, the alkali metal-containing compound, the alkaline earth metal, and the alkaline earth metal-containing compound used to a total amount of the aliphatic diamine used is 1.0 mol% or greater and 4.0 mol% or less.

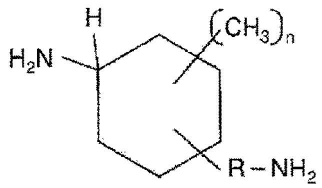

(1)

In Formula (1) above, R represents a single bond or an unsubstituted aliphatic or alicyclic alkylene group having 1 to 8 carbon atoms, and n represents an integer from 0 to 5.

Since the method for isomerizing the aliphatic diamine of the present embodiment includes the isomerization step having the above constitution, it is possible to generate an active species of an isomerization catalyst. As a result, the isomerization reaction of the aliphatic diamine can be easily performed without undergoing a complicated multi-step process as compared with the known technique. In addition, in the isomerization, setting the ratio of the total amount of the aliphatic diamine used and the total amount of the alkali metal, the alkali metal-containing compound, the alkaline earth metal, and the alkaline earth metal-containing compound (hereinafter, the alkali metal, alkali metal-containing compound, alkaline earth metal, and alkaline earth metal-containing compound are also collectively referred to as "alkali metal and the like") used within the above range allows the isomerization reaction of the aliphatic diamine to proceed while suppressing reactions other than the isomerization reaction. As a result, the isomerization reaction of the aliphatic diamine can be performed in a high yield without undergoing a complicated multi-step process as compared with the known technique.

[Isomerization step]

The isomerization step is a step of isomerizing an aliphatic diamine represented by Formula (1) above in the presence of: an imine compound obtained by dehydration condensation of the aliphatic diamine represented by Formula (1) above and an aldehyde and/or a ketone; and at least one selected from the group consisting of an alkali metal, an alkali metal-containing compound, an alkaline earth metal, and an alkaline earth metal-containing compound.

In the present specification, "isomerizing" refers to changing one cis-trans isomer to another cis-trans isomer with respect to the aliphatic diamine represented by Formula (1) above. In more detail, this means changing one cis-trans isomer to another cis-trans isomer by altering a steric configuration (cis-trans configuration) of the amino group directly bonded to a cycloalkane ring in the aliphatic diamine. For example, regarding the aliphatic diamine in which n is 0 in

Formula (1) above, isomerization includes conversion of the cis isomer of the aliphatic diamine into the trans isomer or conversion of the trans isomer of the aliphatic diamine into the cis isomer. Alternatively, regarding the aliphatic diamine in which n is 1 in Formula (1) above which has an amino group bonded to the carbon atom at the 1-position, a methyl group bonded to the carbon atom at the 3-position, and an aminoalkyl group represented by $-R-NH_2$ bonded to the carbon atom at the 5-position of the cyclohexane ring in which the methyl group bonded to the carbon atom at the 3-position and the aminoalkyl group bonded to the carbon atom at the 5-position have a cis configuration, isomerization includes conversion of the (1,3-trans; 1,5-trans) isomer of the aliphatic diamine into the (1,3-cis; 1,5-cis) isomer or conversion of the (1,3-cis; 1,5-cis) isomer of the aliphatic diamine into the (1,3-trans; 1,5-trans) isomer.

Further, the term "isomerizing" includes, when the aminoalkyl group represented by $-R-NH_2$ in Formula (1) above is an aminoalkyl group containing a cycloalkylene group in R, and the amino group of the aminoalkyl group is an aminoalkyl group directly bonded to a cycloalkane ring in R, that is, when the compound represented by Formula (1) is a compound having two aminocycloalkyl groups, changing the steric configuration of each of two amino groups directly bonded to a cycloalkane ring changes the proportions of geometric isomers. The phrase "changing the proportions of geometric isomers" includes, for example, changing the proportions of a cis-cis isomer, a cis-trans isomer, and a trans-trans isomer in diaminodicycloalkylalkylene.

The isomerization step is not particularly limited as long as it is a step of isomerizing an aliphatic diamine represented by Formula (1) above in the presence of an imine compound obtained by dehydration condensation of the aliphatic diamine represented by Formula (1) above and an aldehyde and/or a ketone, and an alkali metal and the like. That is, in the isomerization, the aliphatic diamine represented by Formula (1) above is isomerized by allowing the imine compound, the alkali metal and the like, and the aliphatic diamine represented by Formula (1) above to coexist in the reaction system.

The isomerization reaction temperature in the isomerization step is preferably 10°C or higher and 200°C or lower, more preferably 80°C or higher and 150°C or lower, further preferably 100°C or higher and 140°C or lower, and particularly preferably 100°C or higher and 130°C or lower. When the isomerization reaction temperature is 10°C or higher, an isomerization reaction tends to be able to proceed more efficiently. When the isomerization reaction temperature is 200°C or lower, a side reaction such as a decomposition reaction and a polymerization reaction can be suppressed and co-production of low-boiling point products and high-boiling point products can be reduced, and thus the recovery rate of aliphatic diamine tends to be further improved. Particularly, when the isomerization reaction temperature is controlled to be 100°C or higher and 140°C or lower, more excellent yield and reaction rate tend to be successfully obtained. The isomerization reaction temperature may be within a range obtained by optionally combining the upper and lower limits described above.

The isomerization reaction time varies depending on the usage amount of each component, the reaction conditions, the target isomer composition, and the like, and is preferably 0.5 hours or longer and 8.0 hours or shorter, more preferably 1.0 hours or longer and 7.0 hours or shorter, and even more preferably 2.0 hours or longer and 6.5 hours or shorter. When the isomerization reaction time is within the above range, the isomerization reaction of the aliphatic diamine tends to proceed sufficiently while further reducing the co-production of low-boiling point products and high-boiling point products. The isomerization reaction time may be within a range obtained by optionally combining the upper and lower limits described above.

The isomerization reaction can be performed either in the presence or absence of a solvent. Examples of the solvent that can be used include, but are not particularly limited to, a solvent inert to an aliphatic diamine, an aldehyde and a ketone. Examples of such a solvent include, but are not particularly limited to, aromatic solvents such as benzene, toluene, and xylene; ether solvents such as diethyl ether and tetrahydrofuran; and hydrocarbon solvents such as hexane or heptane. Of them, in order to promote the isomerization reaction more efficiently, a solvent having a boiling point equal to or lower than the isomerization reaction temperature is preferable.

The isomerization reaction atmosphere is not particularly limited, and for example, an atmosphere not containing air or active hydrogen such as water and an alcohol is preferable. If such an atmosphere is employed, an active species of an isomerization catalyst, which is produced by coexistence of the imine compound and the alkali metal and the like, is rarely inactivated and the reaction efficiency tends to be further improved. Particularly, from the viewpoint of suppressing inactivation by the reaction of the active species of a catalyst with water possibly present in the reaction system, the water content in the reaction system is preferably controlled to be 1000 ppm or less. As a convenient method for preventing contamination with moisture, air, or the like, an isomerization reaction is preferably performed in an atmosphere of an inert gas such as nitrogen gas or argon gas.

In the isomerization step, bubbling is preferably performed by supplying an inert gas in the reaction system. Consequently, the isomerization reaction tends to be more efficiently promoted.

In the isomerization step, the aliphatic diamine constituting the imine compound and the aliphatic diamine to be isomerized may be the same or different. From the viewpoint that the isomerization reaction of the aliphatic diamine can be performed more simply and in a higher yield, the aliphatic diamine constituting the imine compound and the aliphatic diamine to be isomerized are preferably the same in the isomerization step.

The isomerization step, the aliphatic diamine, the imine compound, and the alkali metal and the like will be more specifically described below.

[Dehydration condensation step]

The imine compound used in the isomerization step may be an imine compound generated in the reaction system of the isomerization method of the present embodiment or an imine compound added separately in the reaction system. Therefore, the isomerization method of the present embodiment may include dehydration condensation step in which the aliphatic diamine and the aldehyde and/or ketone are mixed and subjected to dehydration condensation to obtain the imine compound in the reaction system before the isomerization and/or during the isomerization. That is, the isomerization method of the present embodiment may include the dehydration condensation step before the isomerization step, or the isomerization step may also serve as the dehydration condensation step.

Examples of the case where the isomerization step also serves as the dehydration condensation step include a case where the dehydration condensation reaction of the aliphatic diamine and the aldehyde and/or ketone and the isomerization reaction of the aliphatic diamine are allowed to simultaneously proceed by simultaneously adding all of the aliphatic diamine represented by Formula (1) above, the aldehyde and/or ketone, and the alkali metal and the like into the reaction system.

Examples of the case where the dehydration condensation step is included before the isomerization step include a case where the aliphatic diamine represented by Formula (1) above and the aldehyde and/or ketone are added to a first reaction system to cause a dehydration condensation reaction, thereby obtaining the imine compound, then the imine compound is isolated from the first reaction system, and then the obtained imine compound, the aliphatic diamine, and the alkali metal and the like are added to a second reaction system different from the first reaction system to cause an isomerization reaction of the aliphatic diamine. Here, the aliphatic diamines added to the first reaction system and the second reaction system may be the same or different, and are preferably the same.

Examples of another case where the dehydration condensation step is included before the isomerization step include a case where the aliphatic diamine represented by Formula (1) above and the aldehyde and/or ketone are added to a reaction system to cause a dehydration condensation reaction, thereby obtaining the imine compound, and then the aliphatic diamine and the alkali metal and the like are further added to the reaction system while the imine compound remains in the reaction system to cause the isomerization reaction of the aliphatic diamine. Here, the aliphatic diamine added first and the aliphatic diamine added subsequently may be the same or different, but are preferably the same.

Examples of still another case where the dehydration condensation step is included before the isomerization step include a case where the aldehyde and/or ketone and the aliphatic diamine represented by Formula (1) above in an excess amount with respect to the total amount of the aldehyde and ketone are added to the reaction system to cause a dehydration condensation reaction, thereby obtaining the imine compound, and then the alkali metal and the like are further added to the reaction system while the imine compound and the aliphatic diamine remain in the reaction system to cause the isomerization reaction of the aliphatic diamine.

When the imine compound obtained by the dehydration condensation of the aliphatic diamine represented by Formula (1) and the aldehyde and/or ketone is available as a reagent, the isomerization method of the present embodiment need not include the dehydration condensation. The imine compound obtained as a reagent can also be used without any particular purification.

The isomerization method of the present embodiment preferably includes the dehydration condensation before the isomerization, and the isomerization and the dehydration condensation are performed in the same reaction system. According to such an aspect, since it is not necessary to isolate the imine compound obtained by the dehydration condensation of the aliphatic diamine and the aldehyde and/or the ketone, and the side reaction can be suppressed, as compared with the known technique, the isomerization reaction of the aliphatic diamine can be performed more easily and in a higher yield without undergoing a complicated multi-step process. Furthermore, in such an aspect, the aliphatic diamine constituting the imine compound and the aliphatic diamine to be isomerized are preferably the same. The "aliphatic diamine constituting an imine compound" refers to an aliphatic diamine including a partial structure derived from an aliphatic diamine contained in the imine compound, that is, an aliphatic diamine used to obtain the imine compound.

Furthermore, when the isomerization method of the present embodiment includes the dehydration condensation, an aldehyde or ketone that is industrially easily and inexpensively available can be used as a catalyst raw material without using an expensive noble metal catalyst or the like. As a result, it becomes possible to perform the isomerization of the aliphatic diamine industrially advantageously, and thus the industrial significance is extremely high.

The dehydration condensation temperature in the dehydration condensation step is preferably 10°C or higher and 200°C or lower, more preferably 80°C or higher and 150°C or lower, further preferably 100°C or higher and 140°C or lower, and particularly preferably 100°C or higher and 130°C or lower. When the dehydration condensation temperature is 10°C or higher, the dehydration condensation reaction tends to be able to proceed more efficiently. When the dehydration condensation temperature is 200°C or lower, a side reaction such as a decomposition reaction and a polymerization

reaction can be suppressed and co-production of low-boiling point products and high-boiling point products can be reduced, and thus the recovery rate of the aliphatic diamine tends to be further improved. Particularly, when the dehydration condensation temperature is controlled to be 100°C or higher and 140°C or lower, more excellent yield and reaction rate tend to be successfully obtained. The dehydration condensation temperature may be within a range obtained by optionally combining the upper and lower limits described above.

The dehydration condensation time varies depending on the usage amount of each component, the reaction conditions, and the like, and is preferably 0.1 hours or longer and 3.0 hours or shorter, more preferably 0.2 hours or longer and 2.0 hours or shorter, and even more preferably 0.3 hours or longer and 1.0 hour or shorter. When the dehydration condensation time is within the above range, the isomerization reaction of the aliphatic diamine tends to proceed sufficiently while further reducing the co-production of low-boiling point products and high-boiling point products. The dehydration condensation time may be within a range obtained by optionally combining the upper and lower limits described above.

The dehydration condensation step can be performed in the presence or absence of a catalyst. The dehydration condensation step can be performed in the presence or absence of a solvent. Examples of the solvent that can be used include, but are not particularly limited to, a solvent inert to an aliphatic diamine, an aldehyde, and a ketone. Examples of such a solvent include, but are not particularly limited to, aromatic solvents such as benzene, toluene, and xylene; ether solvents such as diethyl ether and tetrahydrofuran; and hydrocarbon solvents such as hexane and heptane.

Examples of the dehydration condensation include, but are not particularly limited to, a method of azeotropically dehydrating the components in a benzene solvent using a Dean-Stark apparatus. According to such a method, the imine compound can be easily obtained. When the dehydration condensation reaction is performed in the absence of a solvent, the dehydration condensation can easily proceed by removing water from the system through a distillation operation or the like.

When the isomerization method of the present embodiment includes the dehydration condensation step before the isomerization step, and the isomerization step and the dehydration condensation step are performed in the same reaction system, the isomerization method of the present embodiment may include removing water generated by the dehydration condensation after the dehydration condensation step and before the isomerization step.

Examples of such water removal step include, but are not particularly limited to, a method in which water is removed under reduced pressure conditions of, for example, 3 torr or greater and 300 torr or less by maintaining a temperature of, for example, 80°C or higher and 200°C or lower (1 torr= 1.33 mbar).

[Raw material ratio]

In the isomerization method of the present embodiment, the ratio of the total amount of the alkali metal, the alkali metal-containing compound, the alkaline earth metal, and the alkaline earth metal-containing compound used to the total amount of the aliphatic diamine used (100 mol%) is 0.5 mol% or greater and 6.0 mol% or less. By setting the ratio of the total amount of the aliphatic diamine used and the total amount of alkali metal and the like used to be within the above range, the isomerization reaction can proceed more rapidly and smoothly while suppressing side reactions such as polymerization reaction of the aliphatic diamine, and thus the isomerization reaction of the aliphatic diamine can proceed sufficiently while improving the yield of the target isomer. As a result, the isomerization reaction of the aliphatic diamine can be easily performed in a high yield without undergoing a complicated multi-step process as compared with the known technique.

In the present specification, the "total amount of aliphatic diamine used" means the total amount of substance of the aliphatic diamine used in the isomerization method of the present embodiment. More specifically, the "total amount of aliphatic diamine used" is the total of the amount of aliphatic diamine used constituting the imine compound and the amount of aliphatic diamine used to be isomerized. Regarding the phrase "the amount of aliphatic diamine used constituting the imine compound", in a case where a moiety derived from an aliphatic diamine is contained in n positions (n is a natural number) per molecule of the imine compound, that is, in a case where one molecule of an imine compound is obtained by dehydration condensation of n molecules of the aliphatic diamine, and an aldehyde and/or a ketone, the amount of the aliphatic diamines used is n times the amount of the imine compound used.

Regarding the isomerization step and the dehydration condensation step, the "total amount of aliphatic diamines used" will be specifically described. In the method in which the dehydration condensation reaction of the aliphatic diamine and the aldehyde and/or ketone and the isomerization reaction of the aliphatic diamine proceed simultaneously by simultaneously adding $N_1$ mol of aliphatic diamine, the aldehyde and/or ketone, and the alkali metal and the like into the reaction system, the total amount of the aliphatic diamine used is $N_1$ mol.

In the method in which the aliphatic diamine and the aldehyde and/or ketone are added to a first reaction system to cause the dehydration condensation reaction, thereby obtaining an imine compound, then the imine compound is isolated from the first reaction system, and then $N_2$ mol of the obtained imine compound, $N_3$ mol of the aliphatic diamine, and the alkali metal and the like are added to a second reaction system different from the first reaction system to cause the isomerization reaction of the aliphatic diamine, the total amount of the aliphatic diamine used is $(nN_2 + N_3)$ mol. It is

assumed that a moiety derived from the aliphatic diamine is contained in n positions (n is a natural number) per molecule of the imine compound.

In the method in which $N_4$ mol of the aliphatic diamine and the aldehyde and/or ketone are added to the reaction system to cause the dehydration condensation reaction, thereby obtaining an imine compound, and then $N_5$ mol of the aliphatic diamine and alkali metal and the like are further added to the reaction system while the imine compound remains in the reaction system to cause the isomerization reaction of the aliphatic diamine, the total amount of the aliphatic diamine used is ($N_4$ + $N_5$) mol.

In the method in which the aldehyde and/or the ketone and $N_6$ mol of the aliphatic diamine in an excess amount with respect to the total amount of the aldehyde and the ketone are added to the reaction system to cause the dehydration condensation reaction, thereby obtaining the imine compound, and then the alkali metal and the like are further added to the reaction system while the imine compound and the aliphatic diamine remain in the reaction system to cause the isomerization reaction of the aliphatic diamine, the total amount of the aliphatic diamine used is $N_6$ mol.

In the method for allowing the isomerization reaction of the aliphatic diamine to proceed by simultaneously adding all of $N_7$ mol of a commercially available imine compound, the alkali metal and the like, and $N_8$ mol of the aliphatic diamine to the reaction system, the total amount of the aliphatic diamine used is ($nN_7$ + $N_8$) mol. It is assumed that a moiety derived from the aliphatic diamine is contained in n positions (n is a natural number) per molecule of the imine compound.

In the isomerization method of the present embodiment, the ratio of the total amount of the alkali metal and the like used to the total amount of the aliphatic diamine used (100 mol%) is preferably 1.0 mol% or greater and 5.0 mol% or less, and more preferably 1.5 mol% or greater and 4.0 mol% or less. By setting the ratio of the total amount of the aliphatic diamine used and the total amount of alkali metal and the like used to be within the above range, the isomerization reaction can proceed more rapidly and smoothly while further suppressing side reactions such as polymerization reaction of the aliphatic diamine. Therefore, the isomerization reaction of the aliphatic diamine can proceed sufficiently while further improving the yield of the target isomer. As a result, the isomerization reaction of the aliphatic diamine can be easily performed in a higher yield without undergoing a complicated multi-step process as compared with the known technique. The above ratio may be within a range obtained by optionally combining the upper and lower limits described above.

In the isomerization method of the present embodiment, the ratio of the total amount of the aldehyde and ketone used to the total amount of the aliphatic diamine used (100 mol%) is preferably 3.5 mol% or greater and 15.0 mol% or less, more preferably 4.0 mol% or greater and 10.0 mol% or less, and still more preferably 4.5 mol% or greater and 8.0 mol% or less. By setting the ratio of the total amount of the aliphatic diamine used and the total amount of the aldehyde and ketone used to be within the above range, a more excellent reaction rate of the isomerization reaction tends to be obtained. In this way, the isomerization method of the present embodiment can change the isomer composition ratio significantly. The above ratio may be within a range obtained by optionally combining the upper and lower limits described above.

In the present specification, the "total amount of the aldehyde and ketone used" means the total amount of substances of the aldehyde and ketone used in the isomerization method of the present embodiment. More specifically, the "total amount of the aldehyde and ketone used" is the total amount of the aldehyde used constituting the imine compound and the amount of the ketone used constituting the imine compound. The "aldehyde constituting the imine compound" and the "ketone constituting the imine compound" respectively mean a moiety derived from the aldehyde and a moiety derived from the ketone contained in the imine compound, that is, the aldehyde and the ketone used to obtain the imine compound. Regarding the phrase "the amount of the aldehyde and ketone used constituting the imine compound", in a case where a moiety derived from the aldehyde and/or ketone is contained in n positions (n is a natural number) per molecule of the imine compound, that is, in a case where one molecule of an imine compound is obtained by dehydration condensation of n molecules of an aldehyde and/or a ketone and an aliphatic diamine, the amount of the aldehyde and ketone used is n times the amount of the imine compound used.

In the isomerization method of the present embodiment, the ratio of the total amount of the aldehyde and ketone used to the total amount of the alkali metal and the like used is preferably, by substance amount ratio, 0.9 or greater and 6.0 or less, more preferably 1.0 or greater and 5.0 or less, and still more preferably 1.1 or greater and 4.0 or less. By setting the ratio of the total amount of the alkali metal and the like used and the total amount of aldehyde and ketone used to be within the above range, the isomerization reaction tend to proceed more rapidly and smoothly while further suppressing side reactions such as polymerization reaction of the aliphatic diamine, and thus the isomerization reaction of the aliphatic diamine tends to be able to proceed sufficiently while further improving the yield of the target isomer. As a result, the isomerization reaction of the aliphatic diamine can be easily performed in a higher yield without undergoing a complicated multi-step process as compared with the known technique. The above ratio may be within a range obtained by optionally combining the upper and lower limits described above.

[Aliphatic diamine]

In the present specification, the "aliphatic diamine" refers to a compound represented by the following Formula (1).

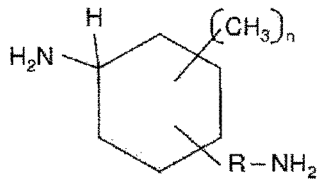

(1)

In Formula (1) above, R represents a single bond or an unsubstituted aliphatic or alicyclic alkylene group having 1 to 8 carbon atoms, and n represents an integer from 0 to 5.

The aliphatic diamine is a substrate to be isomerized, and is also used to form the imine compound by the dehydration condensation with an aldehyde and/or a ketone to form an active species of an isomerization catalyst for the aliphatic diamine. The aliphatic diamines can be used alone or in combination of two or more. In addition, the aliphatic diamine constituting the imine compound and the aliphatic diamine to be isomerized may be the same or different. From the viewpoint that the isomerization reaction of the aliphatic diamine can be performed more simply and in a higher yield, the aliphatic diamine constituting the imine compound and the aliphatic diamine to be isomerized are preferably the same in the isomerization step.

In the isomerization method of the present embodiment, the aliphatic diamine preferably includes an aliphatic diamine represented by Formula (1A) below.

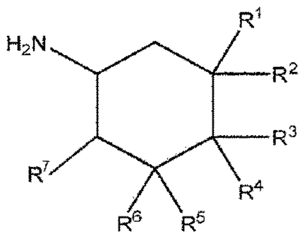

(1 A)

In Formula (1A) above, any one of $R^1$, $R^2$, $R^3$, $R^4$, $R^5$, $R^6$, and $R^7$ is an aminoalkyl group represented by $-R-NH_2$, and the others of $R^1$, $R^2$, $R^3$, $R^4$, $R^5$, $R^6$, and $R^7$ each independently represent a hydrogen atom or a methyl group, and R represents a single bond or an unsubstituted aliphatic or alicyclic alkylene group having 1 to 8 carbon atoms.

According to such an aspect, the isomerization reaction tends to proceed more rapidly and smoothly while further suppressing side reactions such as polymerization reaction of the aliphatic diamine, and thus the isomerization reaction of the aliphatic diamine tends to be able to proceed sufficiently while further improving the yield of the target isomer. From the same perspective, in the isomerization method of the present embodiment, the aliphatic diamine is more preferably the aliphatic diamine represented by Formula (1A) above. In Formula (1A) above, in $R^1$, $R^2$, $R^3$, $R^4$, $R^5$, $R^6$, and $R^7$, the number of methyl groups is 0 or greater and 5 or less, preferably 0 or greater and 4 or less, and more preferably 0 or greater and 3 or less.

In Formulae (1) and (1A) above, R represents a single bond or an unsubstituted aliphatic or alicyclic alkylene group having 1 to 8 carbon atoms. In the present specification, the "unsubstituted aliphatic alkylene group" includes not only a linear alkylene group but also a branched alkylene group. Further, the "unsubstituted alicyclic alkylene group" means a divalent saturated hydrocarbon group having a cycloalkane ring, and includes a divalent saturated hydrocarbon group in which an alkyl group is bonded to a cycloalkane ring. Also, the "unsubstituted alkylene group" means an alkylene group having no heteroatom, and does not exclude an alkylene group having a branched chain. The hetero atom includes, for example, an oxygen atom, a nitrogen atom, a phosphorus atom, and a sulfur atom. R is an unsaturated or saturated alkylene group, and is preferably a saturated alkylene group. The unsubstituted alkylene group is, for example, a linear or branched alkylene group consisting only of a carbon atom and a hydrogen atom.

Examples of the unsubstituted aliphatic alkylene group having 1 to 8 carbon atoms represented by R include, but are not particularly limited to, a methylene group and an ethylene group, and a linear or branched propylene group, butylene group, pentylene group, hexylene group, and heptylene group.

The unsubstituted aliphatic alkylene group represented by R preferably has 1 to 6 carbon atoms, and more preferably has 1 to 4 carbon atoms. According to such an aspect, the isomerization reaction of the aliphatic diamine tends to be able to proceed sufficiently while further improving the yield of the target isomer.

Examples of the unsubstituted alicyclic alkylene group having 1 to 8 carbon atoms represented by R include a divalent group represented by -R'-R"-R'-. Here, R's are each independently a single bond or an aliphatic alkylene group, preferably a single bond, a methylene group, or an ethylene group, more preferably a single bond or a methylene group, and still more preferably a methylene group. R" is divalent cycloalkane, and is preferably selected from the group

consisting of a cyclopropylene group, a cyclobutylene group, a cyclopentylene group, a cyclohexylene group, a cycloheptylene group, and a group in which an alkyl group is bonded to the cycloalkane ring in these groups. R" is more preferably a cyclopentylene group, a cyclohexylene group, or a group in which one methyl group or ethyl group is bonded to the cycloalkane ring in these groups. The sum of the number of carbon atoms of the divalent group represented by -R'-R"-R'- is 1 to 8.

Examples of the unsubstituted alicyclic alkylene group having 1 to 8 carbon atoms represented by R include, but are not particularly limited to, a cyclopropylene group, a cyclobutylene group, a cyclopentylene group, a cyclohexylene group, and a cycloheptylene group; a methylene-cyclopropylene group, a methylene-cyclobutylene group, a methylene-cyclopentylene group, and a methylene-cyclohexylene group; a cyclopropylene-methylene group, a cyclobutylene-methylene group, a cyclopentylene-methylene group, and a cyclohexylene-methylene group; a methylene-cyclopropylene-methylene group, a methylene-cyclobutylene-methylene group, and a methylene-cyclopentylene-methylene group; an ethylene-cyclopropylene group, an ethylene-cyclobutylene group, and an ethylene-cyclopentylene group; a cyclopropylene-ethylene group, a cyclobutylene-ethylene group, and a cyclopentylene-ethylene group; a propylene-cyclopropylene group, a propylene-cyclobutylene group, a cyclopropylene-propylene group, and a cyclobutylene-propylene group; an ethylene-cyclopropylene-methylene group, an ethylene-cyclobutylene-methylene group, a methylene-cyclopropylene-ethylene group, and a methylene-cyclobutylene-ethylene group; and a group in which an alkyl group is bonded to the cycloalkane ring in these groups as long as the number of carbon atoms of the alkylene group is 8 or less.

The unsubstituted alicyclic alkylene group represented by R preferably has 3 to 8 carbon atoms, and more preferably has 5 to 8 carbon atoms. According to such an aspect, the isomerization reaction of the aliphatic diamine tends to be able to proceed sufficiently while further improving the yield of the target isomer.

The aminoalkyl group represented by $-R-NH_2$ is not particularly limited as long as the alkylene group represented by R is the alkylene group exemplified above, and examples thereof include an amino group, an aminocyclohexylmethyl group ($-CH_2-C_6H_{10}-NH_2$), an aminocyclohexylethyl group ($-C_2H_4-C_6H_{10}-NH_2$), an amino methyl cyclohexylmethyl group ($-CH_2-C_6H_9(CH_3)-NH_2$), an aminomethyl group ($-CH_2-NH_2$), and an aminoethyl group ($-C_2H_4-NH_2$). When R is an alicyclic alkylene group, in the aminoalkyl group represented by $-R-NH_2$, the amino group is preferably directly bonded to the cycloalkane ring.

Examples of the aliphatic diamine include, but are not particularly limited to, cyclohexanediamine, 1,3,3-trimethyl-1-(aminomethyl) aminocyclohexane, diaminodicyclohexylmethane, dimethyldiaminodicyclohexylmethane, and methylcyclohexanediamine.

Examples of the cyclohexanediamine include, but are not particularly limited to, 1,2-cyclohexanediamine, 1,3-cyclohexanediamine, and 1,4-cyclohexanediamine. Of them, 1,4'-cyclohexanediamine is preferable from the viewpoint of more effectively and reliably exhibiting the effects of the present invention. The cyclohexanediamines can be used alone or in combination of two or more.

Examples of the 1,3,3-trimethyl-1-(aminomethyl) aminocyclohexane include, but are not particularly limited to, 1,3,3-trimethyl-1-(aminomethyl)-5-aminocyclohexane, 1,3,3-trimethyl-1-(aminomethyl)-4-aminocyclohexane, 1,3,3-trimethyl-1-(aminomethyl)-6-aminocyclohexane, and 1,3,3-trimethyl-1-(aminomethyl)-2-aminocyclohexane. Of them, 1,3,3-trimethyl-1-(aminomethyl)-5-aminocyclohexane, that is, isophoronediamine is preferable from the viewpoint of more effectively and reliably exhibiting the effects of the present invention. The 1,3,3-trimethyl-1-(aminomethyl) aminocyclohexanes can be used alone or in combination of two or more.

Examples of the diaminodicyclohexylmethane include, but are not particularly limited to, 2,2'-diaminodicyclohexylmethane, 2,3'-diaminodicyclohexylmethane, 2,4'-diaminodicyclohexylmethane, 3,3'-diaminodicyclohexylmethane, 3,4'-diaminodicyclohexylmethane, and 4,4'-diaminodicyclohexylmethane. Of them, 4,4'-diaminodicyclohexylmethane is preferable from the viewpoint of more effectively and reliably exhibiting the effects of the present invention. The diaminodicyclohexylmethanes can be used alone or in combination of two or more of the above.

Examples of the dimethyldiaminodicyclohexylmethane include, but not particularly limited to, 2,2'-diamino-3,3'-dimethyldicyclohexylmethane, 2,2'-diamino-4,4'-dimethyldicyclohexylmethane, 2,2'-diamino-5,5'-dimethyldicyclohexylmethane, 2,2'-diamino-6,6'-dimethyldicyclohexylmethane, 3,3'-diamino-2,2'-dimethyldicyclohexylmethane, 3,3'-diamino-4,4'-dimethyldicyclohexylmethane, 3,3'-diamino-5,5'-dimethyldicyclohexylmethane, 3,3'-diamino-6,6'-dimethyldicyclohexylmethane, 4,4'-diamino-2,2'-dimethyldicyclohexylmethane, and 4,4'-diamino-3,3'-dimethyldicyclohexylmethane. Of them, 4,4'-diamino-3,3'-dimethyldicyclohexylmethane is preferable from the viewpoint of more effectively and reliably exhibiting the effects of the present invention. The dimethyldiaminodicyclohexylmethanes can be used alone or in combination of two or more of the above.

Examples of the methylcyclohexanediamine include, but not particularly limited to, 1-methyl-2,4-cyclohexanediamine and 2-methyl-1,3-cyclohexanediamine. Of them, a mixture of 1-methyl-2,4-cyclohexanediamine and 2-methyl-1,3-cyclohexanediamine is preferable from the viewpoint of more effectively and reliably exhibiting the effects of the present invention. The methylcyclohexanediamines can be used alone or in combination of two or more of the above.

**In** another preferable aspect of the isomerization method of the present embodiment, the aliphatic diamine includes an aliphatic diamine represented by Formula (1B) below, or is an aliphatic diamine represented by Formula (1B)

below.

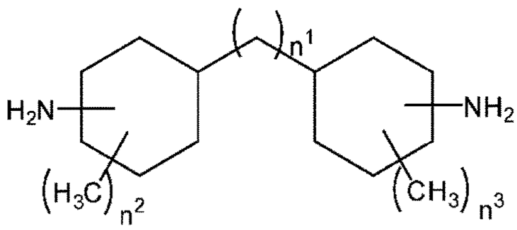

(1 B)

In Formula (1B) above, $n^1$ is an integer from 0 to 2, and $n^2$ is an integer from 0 to 5, $n^3$ is an integer from 0 to 2, and a sum of $n^1$ and $n^3$ is 2 or less.

According to such an aspect, the isomerization reaction tends to proceed more rapidly and smoothly while further suppressing side reactions such as a polymerization reaction of the aliphatic diamine. Therefore, in this aspect, the isomerization reaction of the aliphatic diamine tends to be able to proceed sufficiently while further improving the yield of the target isomer. Further, diisocyanate produced using, as a raw material, the aliphatic diamine obtained according to this aspect is particularly suitable as a raw material for polyurethane.

In Formula (1B) above, $n^1$ is an integer from 0 to 2, preferably 1 or 2, and more preferably 1. $n^2$ is an integer from 0 to 5, preferably an integer from 0 to 2, and more preferably 0 or 1. $n^3$ is an integer from 0 to 2, preferably 0 or 1. When $n^1$, $n^2$, and/or $n^3$ are within the above range, the isomerization of the aliphatic diamine tends to be realized more easily and in a high yield. From the same perspective, it is preferable that the amino group and the methyl group are not bonded to the same carbon atom in Formula (1B).

When $n^1$ is 0, the aliphatic diamine represented by Formula (1B) has an aspect in which two cyclohexane rings are bonded by a single bond. When $n^2$ or $n^3$ is 0, the aliphatic diamine does not include the corresponding methyl group.

In Formula (1B) above, the bonding position of the methyl group and the amino group is not particularly limited, but the amino group is preferably bonded to a 4-position of the cyclohexane ring. In this aspect, the aliphatic diamine represented by Formula (1B) has a structure in which the methyl group is optionally bonded to 4,4'-diaminodicyclohexylalkane. According to such an aspect, the isomerization efficiency of the aliphatic diamine tends to be further improved.

When the aliphatic diamine includes cyclohexanediamine and/or 1,3,3-trimethyl-1-(aminomethyl) aminocyclohexane, the isomerization method of the present embodiment is to change cyclohexanediamine and/or 1,3,3-trimethyl-1-(aminomethyl) aminocyclohexane in a cis isomer to a trans isomer thereof or to change cyclohexanediamine and/or 1,3,3-trimethyl-1-(aminomethyl) aminocyclohexane in a trans isomer to a cis isomer thereof.

When the aliphatic diamine includes diaminodicyclohexylmethane, the isomerization method of the present embodiment is to change proportions of the cis-cis isomer, the cis-trans isomer, and the trans-trans isomer of diaminodicyclohexylmethane.

When the aliphatic diamine includes methylcyclohexanediamine, the isomerization method of the present embodiment is to change methylcyclohexanediamine having a certain stereoisomer structure into an isomer different from the isomer. The phrase "changing methylcyclohexanediamine having a certain stereoisomeric structure into an isomer different from the isomer" means to change the steric configuration (cis-trans configuration) of at least one amino group of the two amino groups directly bonded to the cyclohexane ring of methylcyclohexanediamine.

When the aliphatic diamine includes dimethyldiaminodicyclohexylmethane, the isomerization method of the present embodiment is to change dimethyldiaminodicyclohexylmethane having a certain stereoisomer structure into an isomer different from the isomer. The phrase "changing dimethyldiaminodicyclohexylmethane having a certain stereoisomeric structure into an isomer different from the isomer" means to change the steric configuration (cis-trans configuration) of at least one amino group of the two amino groups directly bonded to the cyclohexane ring of dimethyldiaminodicyclohexylmethane.

Taking isomerization of 4,4'-diamino-3,3'-dimethyldicyclohexylmethane as an example, the isomerization method of the present embodiment can change the proportions of a stereoisomer represented by the following Formula (A), a stereoisomer represented by the following Formula (B), and a stereoisomer represented by the following Formula (C) present. **In** Formulae (A), (B), and (C), the steric configuration of the methyl group may be a cis configuration or a trans configuration, with respect to the amino group. Hereinafter, in dimethyldiaminodicyclohexylmethane, attention is paid to the relationship between the amino group and the aminoalkyl group, and stereoisomers having steric configurations corresponding to Formulae (A), (B), and (C) are referred to as a trans-trans isomer, a cis-trans isomer, and a cis-cis isomer, respectively.

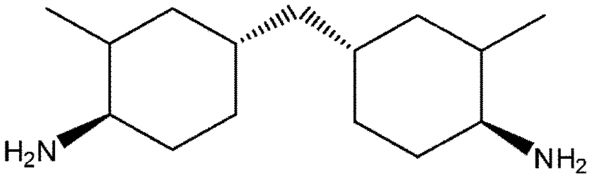

(A)

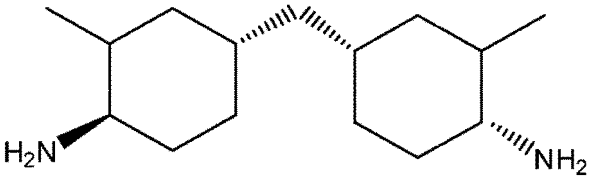

(B)

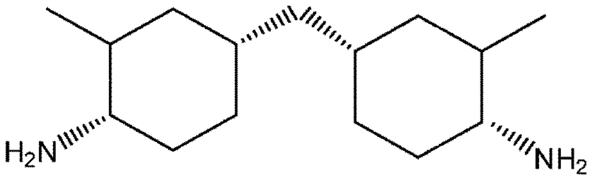

(C)

[Aldehyde]

Examples of the aldehyde used to obtain the imine compound, that is, the aldehyde constituting the imine compound, include, but are not particularly limited to, a compound generally available and having a substituent containing a functional group inert to the alkali metal and the like. Examples of such an aldehyde, although not particularly limited, include, for example, at least one selected from the group consisting of an aliphatic aldehyde represented by the following Formula (2), an aromatic aldehyde represented by the following Formula (3), and an aromatic aldehyde represented by the following Formula (4). By using such a compound, the isomerization yield tends to be further improved. From the same perspective, the aldehydes having an aromatic ring are more preferably used as the aldehyde.

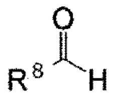

(2)

In Formula (2) above, $R^8$ represents a hydrogen atom or a monovalent substituent selected from the group consisting of a substituted or unsubstituted aliphatic hydrocarbon group and a substituted or unsubstituted alicyclic hydrocarbon group.

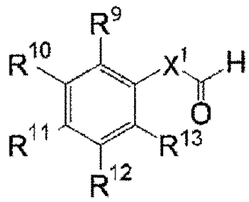

(3)

In Formula (3) above, $R^9$, $R^{10}$, $R^{11}$, $R^{12}$, and $R^{13}$ each independently represent a hydrogen atom or a monovalent group selected from the group consisting of an alkyl group having 1 to 10 carbon atoms, an alkoxy group having 1 to 10 carbon atoms, a phenyl group, and an amino group; and $X^1$ represents a single bond or a divalent alkyl group having 1 to 10 carbon atoms.

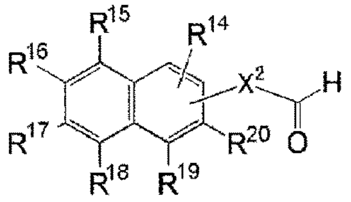

(4)

In Formula (4) above, $R^{14}$, $R^{15}$, $R^{16}$, $R^{17}$, $R^{18}$, $R^{19}$, and $R^{20}$ each independently represent a hydrogen atom or a monovalent group selected from the group consisting of an alkyl group having 1 to 10 carbon atoms, an alkoxy group having 1 to 10 carbon atoms, a phenyl group, and an amino group; and $X^2$ represents a single bond or a divalent alkyl group having 1 to 10 carbon atoms.

Examples of the above aldehyde, although not particularly limited, include, for example, formaldehyde, an aliphatic aldehyde, and an aromatic aldehyde. By using such a compound, the isomer composition ratio can be further greatly changed, and the isomerization yield tends to be further improved. Aldehydes may be used alone or in combination of two or more; and a single aldehyde is preferably used in order to simplify the industrial process.

Examples of the aliphatic aldehyde include, but are not particularly limited to, acetaldehyde, propionaldehyde, 4-isopropylaldehyde, isobutyraldehyde, n-butyraldehyde, n-valeraldehyde, isovaleraldehyde, pivalaldehyde, n-hexylaldehyde, n-heptylaldehyde, n-octylaldehyde, n-nonylaldehyde, n-decylaldehyde, acrolein, methacrolein, 2-methylpentanal, crotonaldehyde, cinnamaldehyde, phenylacetaldehyde, p-methylphenylacetaldehyde, glyoxal, glutaraldehyde, hydroxypivalaldehyde, (+)-citronellal, and (-)-citronellal. Of them, at least one selected from the group consisting of acetaldehyde, isobutyraldehyde, n-decylaldehyde, methacrolein, cinnamaldehyde, and glyoxal, is preferable. By using such a compound, the isomer composition ratio tends to be significantly changed.

The aromatic aldehyde is not particularly limited, and examples thereof include benzaldehyde, 2-methylbenzaldehyde, 3-methylbenzaldehyde, 4-methylbenzaldehyde, 2-ethylbenzaldehyde, 3-ethylbenzaldehyde, 4-ethylbenzaldehyde, 2-propylbenzaldehyde, 3-propylbenzaldehyde, 4-propylbenzaldehyde, 2-isopropylbenzaldehyde, 3-isopropylbenzaldehyde, 4-isopropylbenzaldehyde, 4-biphenylaldehyde, 2-butylbenzaldehyde, 3-butylbenzaldehyde, 4-butylbenzaldehyde, 2-tertiarybutylbenzaldehyde, 3-tertiarybutylbenzaldehyde, 4-tertiarybutylbenzaldehyde, 2-phenylbenzaldehyde, 3-phenylbenzaldehyde, 4-phenylbenzaldehyde, 2,3-dimethylbenzaldehyde, 2,4-dimethylbenzaldehyde, 2,5-dimethylbenzaldehyde, 2,6-dimethylbenzaldehyde, 3,4-dimethylbenzaldehyde, 3,5-dimethylbenzaldehyde, 2,4,5-trimethylbenzaldehyde, 2,4,6-trimethylbenzaldehyde, 2-methoxybenzaldehyde, 3-methoxybenzaldehyde, 4-methoxybenzaldehyde, 2-ethoxybenzaldehyde, 3-ethoxybenzaldehyde, 4-ethoxybenzaldehyde, 1-naphthaldehyde, 2-naphthaldehyde, and 3-naphthaldehyde. Of them, one or more types selected from the group consisting of benzaldehyde, 4-methylbenzaldehyde, 4-ethylbenzaldehyde, 4-isopropylbenzaldehyde, 2,4-dimethylbenzaldehyde, 3,4-dimethylbenzaldehyde, 2,4,5-trimethylbenzaldehyde, 2,4,6-trimethylbenzaldehyde, 4-isobutylbenzaldehyde, and 4-biphenylaldehyde are preferable. By using such a compound, the isomer composition ratio tends to be significantly changed.

(Ketone)

Examples of the ketone used to obtain the imine compound, that is, the ketone constituting the imine compound, include, but are not particularly limited to, a compound generally available and having a substituent containing a functional group inert to the alkali metal and the like. Examples of such a ketone, although not particularly limited, include for example, at least one selected from the group consisting of an aliphatic ketone, an aromatic ketone, an aliphatic-aromatic ketone, and a cyclic ketone. By using such a compound, the isomer composition ratio tends to be significantly changed. The ketone may be used alone or in combination of two or more; and a single aldehyde is preferably used alone in order to simplify the industrial process.

The aliphatic ketone is not particularly limited as long as two aliphatic hydrocarbon groups are bonded to the carbonyl group, and examples thereof include acetone, methyl ethyl ketone, diethyl ketone, methyl propyl ketone, methyl isobutyl ketone, ethyl propyl ketone, ethyl isobutyl ketone, and dipropyl ketone.

The aromatic ketone is not particularly limited as long as two aromatic hydrocarbon groups are bonded to the carbonyl group, and examples thereof include benzophenone.

The aliphatic-aromatic ketone is not particularly limited as long as an aliphatic hydrocarbon and an aromatic hydrocarbon group are bonded to the carbonyl group, and examples thereof include acetophenone.

The cyclic ketone is not particularly limited as long as the carbonyl group constitutes a ring, and examples thereof include cyclohexanone.

Of them, the ketone is preferably one or more selected from the group consisting of a ketone having an aromatic ring and a methyl ethyl ketone, and more preferably a ketone having an aromatic ring. Examples of the ketone having an aromatic ring include the aromatic ketone and the aliphatic-aromatic ketone, and acetophenone is preferable. By using

such a compound, the isomer composition ratio tends to be significantly changed.

[Alkali metal and the like]

In the isomerization method of the present embodiment, the aliphatic diamine is isomerized in the presence of at least one selected from the group consisting of an alkali metal, an alkali metal-containing compound, an alkaline earth metal, and an alkaline earth metal-containing compound. In the isomerization method of the present embodiment, these alkali metal and the like promote a more rapid progression of the isomerization reaction. The alkali metal and the like may be used alone or in combination of two or more.

Of them, in the alkali metal and the like, at least one selected from the group consisting of an alkali metal, an alkali metal hydride, and an alkali metal amide is preferably included, and at least one selected from the group consisting of metallic sodium, sodium amide, and sodium hydride is more preferably included. By using such a substance, the isomerization yield tends to be further improved.

Examples of the alkali metals include, but are not particularly limited to, a metallic sodium, a metallic lithium, and a metallic potassium.

Examples of the alkali metal-containing compounds include, but are not particularly limited to, an alkali metal hydride, an alkali metal amide, a basic oxide, and an alkali metal alkoxide. If such a compound is used, the isomerization yield tends to be further improved. Of them, at least one selected from the group consisting of an alkali metal hydride and an alkali metal amide is preferable. Examples of the alkali metal hydride include, but are not particularly limited to, sodium hydride, lithium hydride, potassium hydride, lithium aluminum hydride, and sodium boron hydride. Examples of the alkali metal amide include, but are not particularly limited to, sodium amide, lithium amide, potassium amide, lithium diisopropylamide, and sodium bis(trimethylsilyl)amide. Furthermore, examples of the basic oxide include, but are not particularly limited to, lithium oxide, sodium oxide, potassium oxide, cesium oxide, magnesium oxide, calcium oxide, strontium oxide, and barium oxide. Furthermore, examples of the alkali metal alkoxide include, but are not particularly limited to, potassium-tert-butoxide.

Examples of the alkaline earth metal include, but are not particularly limited to, metallic magnesium and metallic calcium.

Examples of the alkaline earth metal-containing compound include, but are not particularly limited to, an alkaline earth metal hydride. Examples of the alkaline earth metal hydride include, but are not particularly limited to, calcium hydride and magnesium hydride.

[Other steps]

The isomerization method of the present embodiment may include a purification step such as a catalyst component removal step of removing a catalyst component, a low boiling-point component removal step of removing low boiling-point components, a high boiling-point component removal step for removing high boiling-point components, and an isomer separation step of distilling an isomer of the aliphatic diamine. The "catalyst component" herein specifically refers to the imine compound and the alkali metal and the like. The "low boiling-point components" refer to components having lower boiling points than those of isomers of the aliphatic diamine. The "high boiling-point components" refer to components having higher boiling points than those of isomers of the aliphatic diamine.

The catalyst component removal step, the low boiling-point component removal step, the high boiling-point component removal step, and the isomer separation step may be performed in an arbitrary order.

[Catalyst component removal step]

The catalyst component removal step is a step of removing a catalyst component present in a reaction mixture after the isomerization. Owing to the presence of the catalyst component removal step in the isomerization method of the present embodiment, progress of a side reaction can be further suppressed in the purification step. As the method of removing the catalyst, although it is not particularly limited, for example, thin-film distillation can be used. The catalyst component to be separated herein can be inactivated and then separated or can be separated in an active state. The catalyst component separated in an active state can be used again as a catalyst for the isomerization reaction.

[Low boiling-point component removal step]

The low boiling-point component removal step is a step of removing low boiling-point components having lower boiling points than those of isomers of the aliphatic diamine during and/or after the isomerization. Owing to the presence of the low boiling-point component removal step in the isomerization method of the present embodiment, the yield of the isomer tends to be further improved. The method of removing the low boiling-point components, although not particularly

limited, includes, for example, a method of performing distillation at a temperature equal to or lower than the boiling points of isomers of the aliphatic diamine to remove the low boiling-point components from the reaction mixture.

[High boiling-point component removal step]

The high boiling-point component removal step is a step of removing high boiling-point components having higher boiling points than those of isomers of the aliphatic diamine after the isomerization step. The method of removing the high boiling-point components, although not particularly limited, includes, for example, a method of distilling isomers of the aliphatic diamine from the reaction mixture in the following isomer separation step, and thereafter, removing the high boiling-point components remaining in the reaction mixture.

[Isomer separation step]

The isomer separation step is a step of distilling at least one of isomers of the aliphatic diamine during and/or after the isomerization step. Owing to the presence of the isomer separation step in the isomerization method of the present embodiment, the yield of the isomer tends to be further improved.
As described above, isomers of the aliphatic diamine obtained by the method of the present embodiment can be isolated by a general method such as distillation. When distillation is performed, isomerization is preferably performed while separating isomerized aliphatic diamine. In this manner, the aliphatic diamine including an isomer in a high concentration which is equal to or higher than that in the equivalent composition can be produced.
Distillation conditions such as distillation temperature can be appropriately controlled depending upon target isomer.
Hereinafter, a means for carrying out the isomerization method of the present embodiment will be specifically described; however, the isomerization method of the present embodiment is not limited to the following.
As a first aspect, the isomerization method of the present embodiment can be performed by mixing the imine compound, the alkali metal and the like, and the aliphatic diamine in a reactor. The reactor may include a heating means for heating the reactor, a stirring means for stirring the mixture in the reactor, and a gas supply means for bubbling the mixture in the reactor.
To the reactor, the imine compound, the alkali metal and the like, and the aliphatic diamine may be added in an arbitrary order. A mixture obtained by mixing two components of the imine compound, the alkali metal and the like, and the aliphatic diamine in advance may be added. Alternatively, a mixture obtained by mixing the imine compound, the alkali metal and the like, or the aliphatic diamine, and a solvent may be added.
As an addition means for adding the imine compound, the alkali metal and the like, and the aliphatic diamine, a means which can add these compounds to a reactor at a time for a short time or a means which can continuously add them dropwise over a period of time may be employed.
The reactor may include a gas supply means and a gas exhaust means for adjusting the atmosphere within the reactor. In addition, the reactor may be constituted to reflux a solvent. Furthermore, the reactor may be designed for a batch reaction or a continuous reaction.
As a second aspect, in the isomerization method of the present embodiment, the aliphatic diamine and the aldehyde and/or ketone are supplied to a first reactor to produce the imine compound, and the isomerization reaction may be performed in a second reactor. In this case, the second reactor is connected with the first reactor so that the produced imine compound is supplied. The first reactor and/or the second reactor may include a water removal means (for example, a dean stark apparatus or a distillation apparatus) for removing water from the reaction system. In the case where the aliphatic diamine is used as the amine, the raw materials to be supplied to the second reactor may contain the imine compound and the aliphatic diamine. Other aspects can be configured similarly to the first aspect.
As a third aspect, the imine compound, the alkali metal and the like, and the aliphatic diamine are mixed and reacted in a reactor, and then a reaction product may be purified in a distiller connected with the reactor. In this case, the reactor and the distiller may be integrally configured. Other aspects can be configured similarly to the first aspect.

[Method for producing diisocyanate]

The present invention also provides a method for producing diisocyanate. The method for producing the diisocyanate of the present embodiment includes isomerizing an aliphatic diamine using the method for isomerizing the aliphatic diamine of the present embodiment described above, and obtaining diisocyanate using the isomerized aliphatic diamine as a raw material. The isomerization of the aliphatic diamine can be performed as described above, and preferable aspects are also similar to those described above.
The method for producing diisocyanate using the isomerized aliphatic diamine will be described in detail below.
The method for producing diisocyanate of the present embodiment is not particularly limited as long as it can

produce diisocyanate using, as a raw material, the aliphatic diamine obtained by the isomerization method of the present embodiment. For example, a method in which the aliphatic diamine and phosgene react may be used.

From the viewpoint of controlling the steric configuration of the diisocyanate, it is preferable to select a reaction in which the isomer ratio of the aliphatic diamine used as a raw material is considered not to substantially change, in consideration of the reaction mechanism. According to this aspect, the diisocyanate can be produced while maintaining the isomer ratio of the aliphatic diamine isomerized by the isomerization method of the present embodiment.

Alternatively, in consideration of the reaction mechanism, a reaction that is considered to reverse the steric configuration of the aliphatic diamine used as a raw material may be selected.

As one aspect of the method for producing diisocyanate of the present embodiment, a method in which phosgene is used as a raw material will be described below. According to the following method, the diisocyanate can be produced while the isomer ratio of the aliphatic diamine is substantially maintained.

First, the isomerized aliphatic diamine is dissolved in an appropriate solvent (for example, dichlorobenzene), hydrogen chloride is added thereto, and the mixture is kept at 20 to 60°C for 1 to 4 hours to obtain a chloride. Next, after heating to 120 to 180°C, phosgene is added and reacted for 2 to 8 hours at 120 to 180°C. Here, the reaction may be followed by aging for 1 to 5 hours. Thereafter, hydrogen chloride and phosgene in the system are removed, and then the content is distilled to obtain diisocyanate.

According to the method for producing diisocyanate of the present embodiment described above, since the steric configuration of the aliphatic diamine as a raw material is controlled, diisocyanate having a desired isomer ratio can be easily obtained.

[Method for producing polyurethane]

The present invention also provides a method for producing polyurethane. The method for producing polyurethane according to the present embodiment includes isomerizing an aliphatic diamine using the method for isomerizing the aliphatic diamine of the present embodiment described above, obtaining diisocyanate using the isomerized aliphatic diamine as a raw material, and reacting the diisocyanate obtained in the previous step with polyol to obtain polyurethane. The step of isomerizing an aliphatic diamine and the step of producing diisocyanate can be performed as described above, and preferable aspects are also similar to those described above.

Therefore, the method for producing polyurethane of the present embodiment includes a method for producing diisocyanate of the present embodiment, and polyurethane is produced using the diisocyanate obtained by such a method as a raw material. Hereinafter, a method for producing polyurethane using the diisocyanate obtained by the above-described method as a raw material will be described in detail.

The method for producing polyurethane of the present embodiment is to produce polyurethane by reacting the diisocyanate obtained by the method for producing diisocyanate according to the present embodiment with polyol.

The diisocyanate is not particularly limited as long as it is obtained by the method for producing diisocyanate of the present embodiment, and may be used alone or in combination of two or more.

As the polyol, either a low molecular weight polyol or a high molecular weight polyol may be used, or these may be used in combination. The polyols may be used alone or in combination of two or more.

Examples of the low molecular weight polyol include compounds having two or more hydroxyl groups and having a number average molecular weight of less than 400. Examples of such a polyol include dihydric alcohols such as ethylene glycol, propylene glycol, 1,3-propanediol, 1,4-butylene glycol, 1,3-butylene glycol, 1,2-butylene glycol, 1,5-pentanediol, 1,6-hexanediol, neopentyl glycol, alkanediol (having 7 to 22 carbon atoms), diethylene glycol, triethylene glycol, dipropylene glycol, 3-methyl-1,5-pentanediol, alkane-1,2-diol (having 17 to 20 carbon atoms), 1,3-cyclohexanedimethanol, 1,4-cyclohexanedimethanol, 1,4-cyclohexanediol, hydrogenated bisphenol A, 1,4-dihydroxy-2-butene, 2,6-dimethyl-1-octene-3,8-diol, and bisphenol A; trihydric alcohols such as glycerin and trimethylolpropane; tetrahydric alcohols such as tetramethylolmethane (pentaerythritol), and diglycerin; pentahydric alcohols such as xylitol; hexahydric alcohols such as sorbitol, mannitol, allitol, iditol, dulcitol, altritol, inositol, and dipentaerythritol; heptahydric alcohols such as perseitol; and octahydric alcohols such as sucrose.

Examples of the high molecular weight polyol include compounds having two or more hydroxyl groups and having a number average molecular weight of 400 or more. Examples of the polyol include polyether polyol, polyester polyol, polycarbonate polyol, polyurethane polyol, epoxy polyol, vegetable oil polyol, polyolefin polyol, acrylic polyol, and vinyl monomer-modified polyol.

Examples of the polyether polyol include polypropylene glycol and polytetramethylene ether glycol. The polyether polyol can be obtained, for example, by addition polymerization of an alkylene oxide such as ethylene oxide or propylene oxide using a low molecular weight polyol or polyamine as an initiator. The polyether polyol may contain a random and/or block copolymer of two or more types of alkylene oxides. The polyether polyol may be a ring-opening polymer obtained by cationic polymerization of tetrahydrofuran, or amorphous polyether polyol obtained by copolymerizing a low molecular weight polyol with polymerization units of tetrahydrofuran.

Examples of the polyester polyol include a polycondensate obtained by reacting a low molecular weight polyol (preferably, dihydric alcohol) with a polybasic acid under known conditions. The polyester polyol may be obtained, for example, by ring-opening polymerization of lactones such as ε-caprolactone or γ-valerolactone using a low molecular weight polyol (preferably, a dihydric alcohol) as an initiator.

Examples of the polycarbonate polyol include a ring-opening polymer of ethylene carbonate obtained by using a low molecular weight polyol (preferably, a dihydric alcohol) as an initiator, and amorphous polycarbonate polyol obtained by copolymerizing the low molecular weight dihydric alcohol and the ring-opening polymer.

The polyurethane polyol can be obtained by reacting the polyester polyol, polyether polyol and/or polycarbonate polyol described above with any polyisocyanate. At this time, the blending amount of polyisocyanate is controlled such that the equivalent ratio (OH/NCO) of a hydroxyl group to an isocyanate group is greater than 1.

Examples of the epoxy polyol include those obtained by reacting a low molecular weight polyol with polyfunctional halohydrin such as epichlorohydrin or β-methylepichlorohydrin.

Examples of the vegetable oil polyol include hydroxy group-containing vegetable oils such as castor oil and coconut oil.

Examples of the polyolefin polyol include polybutadiene polyols and partially saponified ethylene-vinyl acetate copolymers.

Examples of the acrylic polyol include a copolymer obtained by copolymerizing a hydroxyl group-containing acrylate and a copolymerizable vinyl monomer.

In the polymerization step of the polyurethane, the diisocyanate and polyol can be reacted in any condition. The polyurethane can be synthesized by a known method such as bulk polymerization, solution polymerization, one-shot method, and prepolymer method.

The type of polyol used, and the reaction conditions of the diisocyanate and polyol can be appropriately adjusted depending on the desired physical properties, and specifically refer to WO 2009/051114 or the like.

In the polymerization step of the polyurethane, a catalyst such as an amine or an organometallic compound may be added as necessary.

Examples of the amines include triethylamine, triethylenediamine, bis-(2-dimethylaminoethyl) ether, N-methylmorpholine, tetraethylhydroxylammonium, imidazole, and 2-ethyl-4-methylimidazole.

Examples of the organometallic compounds include organotin compounds such as tin acetate, tin octylate, tin oleate, tin laurylate, dibutyl tin diacetate, dimethyl tin dilaurate, dibutyl tin dilaurate, dibutyl tin dimercaptide, dibutyl tin maleate, dibutyl tin dilaurate, dibutyl tin dineodecanoate, dioctyl tin dimercaptide, dioctyl tin dilaurylate, and dibutyl tin dichloride; organolead compounds such as lead octanate and lead naphthenate; organonickel compounds such as nickel naphthenate; organocobalt compounds such as cobalt naphthenate; organocopper compounds such as copper octenoate; and organobismuth compounds such as bismuth octylate and bismuth neodecanoate.

One aspect of the method for producing polyurethane of the present embodiment will be described as the production of polyurethane by the prepolymer method.

First, the organometallic compound (for example, an organotin compound) is added to a polyol (for example, polyether polyol), and the mixture is stirred at 60 to 120°C and then dried under reduced pressure at 80 to 150°C for 1 to 4 hours. A diisocyanate melted at 40 to 80°C is added under a nitrogen atmosphere. After the addition, the temperature is raised to 60 to 120°C, and the mixture is stirred for 2 hours with the temperature being maintained, thereby synthesizing a prepolymer. After several minutes of defoaming, dehydrated polyol (for example, a low molecular weight polyol) is added to the prepolymer and stirred for 1 to 10 minutes. After defoaming a reaction product, the reaction product is poured into a mold and held at 80 to 150°C for 10 to 100 hours in a thermostat, whereby polyurethane can be obtained. The obtained polyurethane may be cured at room temperature for 3 to 7 days or more for reaction completion.

According to the method for producing polyurethane of the present embodiment described above, since the steric configuration of the diisocyanate as a raw material is controlled, polyurethane having desired physical properties can be easily obtained. For example, when the diisocyanate having two cycloalkane rings such as bis(isocyanatocyclohexyl) methane or bis(isocyanatomethylcyclohexyl) methane is used as the diisocyanate, the polyurethane having higher rigidity can be obtained by using the diisocyanate having a high trans-trans isomer proportion as a raw material.

[Polyurethane]

The present invention also provides polyurethane. The polyurethane of the present embodiment contains a structure derived from diisocyanate obtained by the production method of the present embodiment. In other words, the polyurethane of the present embodiment is polyurethane obtained by reacting the diisocyanate obtained by the production method according to the present embodiment with polyol. As the polyol, those described above in the method for producing polyurethane of the present embodiment can be used.

Since the polyurethane of the present embodiment has the above constitution, the steric configuration of the moiety derived from diisocyanate is controlled. Therefore, the polyurethane of the present embodiment easily exhibits

desired physical properties.

One preferable aspect of the polyurethane of the present embodiment is polyurethane having a structure derived from a diisocyanate produced using diaminodicyclohexylmethane or dimethyldiaminodicyclohexylmethane isomerized by the isomerization method of the present embodiment as a raw material. Such polyurethane tends to have a high trans-trans isomer proportion of structures derived from diisocyanate and have excellent mechanical properties.

In this aspect, bis(isocyanatocyclohexyl) methane or bis(isocyanatomethylcyclohexyl) methane, which is a raw material of polyurethane, is preferably produced from a diamine having an increased trans-trans isomer proportion.

That is, in one preferable aspect, the polyurethane is obtained by reacting bis(isocyanatocyclohexyl) methane or bis(isocyanatomethylcyclohexyl) methane and polyol, wherein the bis(isocyanatocyclohexyl) methane or bis(isocyanatomethylcyclohexyl) methane is produced from diaminodicyclohexylmethane or dimethyldiaminodicyclohexylmethane with an enhanced trans-trans isomer proportion by the isomerization method of the present embodiment. In this aspect, the trans-trans isomer proportion of diaminodicyclohexylmethane or dimethyldiaminodicyclohexylmethane is preferably 35% or greater, more preferably 40% or greater, still more preferably 50% or greater, and still more preferably 55% or greater. When the trans-trans isomer proportion is within the above range, mechanical properties such as tensile strength and/or hardness of the polyurethane tend to be improved. The upper limit of the trans-trans isomer proportion is not particularly limited, and may be 100%, 90%, 80%, or 70%. The proportion may be within a range obtained by optionally combining the upper and lower limits described above.

The polyurethane of the present embodiment can be used in a wide range of applications such as elastomers (solution-based polyurethane, aqueous polyurethane, powder, thermosetting elastomer, thermoplastic elastomer, spray-molded urethane, elastic fiber, film, sheet, and the like), lenses, synthetic leather, slush powder, elastic molded articles, RIM molded articles, paints, adhesives, sealants, and foams.

Examples

Hereinafter, the present invention will be more specifically described by way of Examples and Comparative Examples. However, the present invention is not limited to these Examples.

[Isomerization of aliphatic diamine]

Isomerization of 4,4'-diaminodicyclohexylmethane and 4,4'-diamino-3,3'-dimethyldicyclohexylmethane was performed.

[Isomer composition]

Isomer compositions (cis-cis/cis-trans/trans-trans proportions) of 4,4'-diaminodicyclohexylmethane and 4,4'-diamino-3,3'-dimethyldicyclohexylmethane were analyzed using gas chromatography equipped with HP1-MS, which is a capillary column available from Agilent.

Of the isomers of 4,4'-diaminodicyclohexylmethane, a trans-trans isomer has the lowest boiling point, a cis-trans isomer has the second lowest boiling point, and a cis-cis isomer has the highest boiling point. In the gas chromatography, the trans-trans isomer, the cis-trans isomer, and the cis-cis isomer were detected sequentially in this order. The cis-cis isomer proportion was calculated in accordance with the equation:

Area value of cis-cis isomer/(area value of cis-cis isomer + area value of cis-trans isomer + area value of trans-trans isomer) $\times$ 100.

The cis-trans isomer proportion was calculated in accordance with the equation:

Area value of cis-trans isomer/(area value of cis-cis isomer + area value of cis-trans isomer + area value of trans-trans isomer) $\times$ 100.

The trans-trans isomer proportion was calculated in accordance with the equation:

Area value of trans-trans isomer/(area value of cis-cis isomer + area value of cis-trans isomer + area value of trans-trans isomer) $\times$ 100.

Further, each isomer of 4,4'-diamino-3,3'-dimethyldicyclohexylmethane (for two amino groups with respect to

each aminomethylcyclohexylmethyl group, isomer with cis-cis configuration; isomer with cis-trans configuration; isomer with trans-trans configuration) also has a different retention time in gas chromatography, and the retention time of the trans-trans isomer is the shortest under the above conditions (for example, refer to Chinese Patent Application Publication No. 102627569). Therefore, the trans-trans isomer proportion was calculated in accordance with the equation:

$$\text{Area value of peak detected first/(sum of area values of respective peaks)} \times 100.$$

The isomer composition (cis/trans proportion) of cyclohexanediamine can be analyzed using gas chromatography equipped with HP1-MS, which is a capillary column available from Agilent. 1,4-Cyclohexanediamine has a lower boiling point in the trans isomer than in the cis isomer, and an isomer detected first by gas chromatography is the trans isomer, and an isomer detected later is the cis isomer. The cis isomer proportion can be calculated in accordance with the equation:

Area value of cis isomer/(area value of cis isomer + area value of trans isomer) $\times$ 100.

The trans isomer proportion can be calculated in accordance with the equation:

$$100 - \text{cis isomer proportion.}$$

In addition, the isomer composition (cis/trans proportion) of 1,3,3-trimethyl-1-(aminomethyl) aminocyclohexane can be analyzed using gas chromatography equipped with CP-Volamine, which is a capillary column available from Valian. In particular, isophoronediamine has a lower boiling point in the trans isomer than in the cis isomer, and an isomer detected first by gas chromatography is the trans isomer, and an isomer detected later is the cis isomer. The cis isomer proportion can be calculated in accordance with the equation:

Area value of cis isomer/(area value of cis isomer + area value of trans isomer) $\times$ 100.

The trans isomer proportion can be calculated in accordance with the equation:

$$100 - \text{cis isomer proportion.}$$

The isomer compositions of methylcyclohexanediamine (proportions of isomer with cis-cis configuration/isomer with trans-cis configuration/isomer with cis-trans configuration/isomer with trans-trans configuration, for the amino group with respect to the methyl group) can be analyzed using gas chromatography equipped with HP1-MS, which is a capillary column available from Agilent. The order in which the isomers are detected by gas chromatography is known in the related art. The proportion of each isomer can be calculated in accordance with the equation:

$$\text{Area value of target isomer/(sum of area values of isomers)} \times 100.$$

[Isomerization yield]

Isomerization yields were calculated by the internal standard method of the above gas chromatography analysis.

Isomerization yield (%) = (total of aliphatic diamine after isomerization reaction)/(sum of aliphatic diamine before isomerization reaction) $\times$ 100

When the isomerization yield is not 100%, it is considered that a high-boiling point product is produced mainly by polymerization of the aliphatic diamine.

[Raw materials]

As 4-methylbenzaldehyde, sodium amide, 4,4'-diaminodicyclohexylmethane, and 4,4'-diamino-3,3'-dimethyldicyclohexylmethane, those available as reagents were used.

Example 1

In a 300 mL flask, 400 g of 4,4'-diaminodicyclohexylmethane (cis-cis isomer: 38.8%, cis-trans isomer: 48.2%, trans-trans isomer: 17.0%) and 14 g of 4-methylbenzaldehyde were weighed and stirred at 120°C for 0.5 hours. After stirring, water removal under reduced pressure was performed at 8 torr and 130°C. After water removal, 2.5 g of sodium amide was added under an argon atmosphere, and an isomerization reaction was performed at normal pressure and 125°C for 6 hours. The isomer proportion after the reaction for 2 hours was cis-cis isomer: 6.4%, cis-trans isomer: 37.5%, trans-trans isomer: 56.1%, the isomer proportion after the reaction for 4 hours was cis-cis isomer: 5.9%, cis-trans isomer: 36.3%, trans-trans isomer: 57.8%, and the isomer proportion after the reaction for 6 hours was cis-cis isomer: 5.7%, cis-trans isomer: 35.9%, trans-trans isomer: 58.4%. The isomerization yield after a lapse of 6 hours was 95.1%. The change in the isomer proportion over time is illustrated in FIG. 1.

Example 2

An isomerization reaction was performed in the same manner as in Example 1 except that the amount of sodium amide used was changed to 2.0 g. The isomer proportion after the reaction for 6 hours was cis-cis isomer: 5.7%, cis-trans isomer: 35.9%, trans-trans isomer: 58.4%. The isomerization yield after a lapse of 6 hours was 96.4%.

Example 3

An isomerization reaction was performed in the same manner as in Example 1 except that the amount of 4-methylbenzaldehyde used was changed to 8.0 g and the amount of sodium amide used was changed to 2.9 g. The isomer proportion after the reaction for 6 hours was cis-cis isomer: 6.4%, cis-trans isomer: 37.4%, trans-trans isomer: 56.2%. The isomerization yield after a lapse of 6 hours was 95.9%.

Comparative Example 1

An isomerization reaction was performed in the same manner as in Example 1 except that the amount of sodium amide used was changed to 5 g. The isomer proportion after the reaction for 6 hours was cis-cis isomer: 5.7%, cis-trans isomer: 35.9%, trans-trans isomer: 58.4%. The isomerization yield after a lapse of 6 hours was 93.3%.

Comparative Example 2

An isomerization reaction was performed in the same manner as in Example 1 except that the amount of 4-methylbenzaldehyde used was changed to 16.2 g and the amount of sodium amide used was changed to 5.9 g. The isomer proportion after the reaction for 6 hours was cis-cis isomer: 5.5%, cis-trans isomer: 35.7%, trans-trans isomer: 58.9%. The isomerization yield after a lapse of 6 hours was 91.5%.

Example 4

In a 300 mL flask, 226 g of 4,4'-diamino-3,3'-dimethyldicyclohexylmethane (trans-trans isomer: 28.9%) and 2.7 g of 4-methylbenzaldehyde were weighed and stirred at 120°C for 0.5 hours. After stirring, water removal under reduced pressure was performed at 8 torr and 130°C. After water removal, 1.0 g of sodium amide was added under an argon atmosphere, and an isomerization reaction was performed at normal pressure and 125°C for 6 hours. The trans-trans isomer proportion after the reaction for 2 hours was 36.1%, the trans-trans isomer proportion after the reaction for 4 hours was 39.5%, and the trans-trans isomer proportion after the reaction for 6 hours was 39.9%. The isomerization yield after a lapse of 6 hours was 96.6%.

Example 5

An isomerization reaction was performed in the same manner as in Example 4 except that the amount of 4-methylbenzaldehyde used was changed to 7.0 g. The trans-trans isomer proportion after the reaction for 6 hours was 41.3%. The isomerization yield after a lapse of 6 hours was 95.4%.

Comparative Example 3

An isomerization reaction was performed in the same manner as in Example 4 except that the amount of 4-methylbenzaldehyde used was changed to 8.4 g and the amount of sodium amide used was changed to 2.5 g. The trans-trans isomer proportion after the reaction for 6 hours was 41.5%. The isomerization yield after a lapse of 6 hours was 92.8%.

Raw material ratios, isomerization yields, and isomer proportions of Examples and Comparative Examples are shown in Tables 1 and 2. In Table 1, the isomer composition (cis-cis/cis-trans/trans-trans proportions) indicates the isomer composition after a lapse of 6 hours from the start of the isomerization reaction, and in Table 2, the trans-trans proportion indicates the proportion after a lapse of 6 hours from the start of the isomerization reaction.

Table 1

| | Example 1 | Example 2 | Example 3 | Comparative Example 1 | Comparative Example 2 |
|---|---|---|---|---|---|
| Alkali metal and the like/aliphatic diamine (mol%) | 3.4 | 2.7 | 3.9 | 6.7 | 7.9 |
| Aldehyde and ketone/aliphatic diamine (mol%) | 6.1 | 6.1 | 3.5 | 6.1 | 7.1 |
| Isomerization yield (%) | 95.1 | 96.4 | 95.9 | 93.3 | 91.5 |
| Trans-trans proportion (%) | 58.4 | 58.4 | 56.2 | 58.4 | 58.9 |
| Cis-trans proportion (%) | 35.9 | 35.9 | 37.4 | 35.9 | 35.7 |
| Cis-cis proportion (%) | 5.7 | 5.7 | 6.4 | 5.7 | 5.5 |

Table 2

| | Example 4 | Example 5 | Comparative Example 3 |
|---|---|---|---|
| Alkali metal and the like/aliphatic diamine (mol%) | 2.6 | 2.6 | 7.7 |
| Aldehyde and ketone/aliphatic diamine (mol%) | 2.3 | 5.8 | 7.0 |
| Isomerization yield (%) | 96.6 | 95.4 | 92.8 |
| Trans-trans proportion (%) | 39.9 | 41.3 | 41.5 |

As shown in Table 1, according to the isomerization methods of Examples and Comparative Examples, it was possible to increase the trans-trans proportion, and to decrease the cis-trans proportion and the cis-cis proportion, and it was found that the isomerization methods of Examples and Comparative Examples were able to easily realize the isomerization of the aliphatic diamine. In addition, in Examples 1 to 3 in which the ratio of the total amount of the alkali metal, the alkali metal-containing compound, the alkaline earth metal, and the alkaline earth metal-containing compound used to the total amount of the aliphatic diamine used is 0.5 mol% or greater and 6.0 mol% or less, it was found that the yield was high as compared with Comparative Examples 1 and 2 in which the ratio was out of the above range. In particular, in Examples 1 to 3, the isomerization yield was 95.0% or greater, and it was found that the isomerization method of the present embodiment can realize the isomerization of the aliphatic diamine in a very high yield.

In addition, as shown in Table 2, it was found that the isomerization of the aliphatic diamine was able to be realized in a high yield in Examples 4 and 5 as compared with Comparative Example 3.

[Production of isocyanate and polyurethane]

A diisocyanate (dicyclohexylmethane-4,4'-diisocyanate) was produced using 4,4'-diaminodicyclohexylmethane as a raw material, and then polyurethane was produced using the obtained diisocyanate as a raw material.

[Isomer composition]

The isomeric composition (cis-cis/cis-trans/trans-trans proportions) of dicyclohexylmethane-4,4'-diisocyanate was analyzed using gas chromatography equipped with HP1-MS, which is a capillary column available from Agilent.

Specifically, the isomer composition was measured by utilizing the fact that each isomer of dicyclohexylmethane-4,4'-diisocyanate was detected in the order of the trans-trans isomer, the cis-trans isomer, and the cis-cis isomer in gas chromatography. The cis-cis isomer proportion was calculated in accordance with the equation:

Area value of cis-cis isomer/(area value of cis-cis isomer + area value of cis-trans isomer + area value of trans-trans isomer) × 100.

The cis-trans isomer proportion was calculated in accordance with the equation:

Area value of cis-trans isomer/(area value of cis-cis isomer + area value of cis-trans isomer + area value of trans-trans isomer) × 100.

The trans-trans isomer proportion was calculated in accordance with the equation:

Area value of trans-trans isomer/(area value of cis-cis isomer + area value of cis-trans isomer + area value of trans-trans isomer) × 100.

[Raw materials]

4,4'-diaminodicyclohexylmethane was prepared by distilling the reaction mixture obtained in Example 1. Specifically, 4,4'-diaminodicyclohexylmethane (hereinafter, referred to as "PACM-t") having a purity of 98.6%, a cis-cis isomer of 5.6%, a cis-trans isomer of 36.7%, and a trans-trans isomer of 57.6% was used.
Other raw materials available as reagents were used.

Example 6: Production of dicyclohexylmethane-4,4'-diisocyanate

To a 5 L four-necked flask equipped with a Dimroth condenser, 300.0 g of the above PACM-t and 3930 g of o-dichlorobenzene (available from Kanto Chemical Co., Inc.) were added. Thereafter, 213.0 g of hydrogen chloride (available from Toagosei Co., Ltd.) was added while being stirred with a mechanical stirrer at a rotation speed of 250 rpm. Chloride of white slurry was obtained by holding at an internal temperature of 21 to 40°C for 2 hours and 1 minute. Next, after raising the flask internal temperature to 149°C over 1 hour and 17 minutes, 667.6 g of phosgene (available from Showa Denko KK) was introduced and reacted at an internal temperature of 148 to 153°C for 4 hours and 52 minutes. Subsequently, after aging at 149 to 151°C for 2 hours, nitrogen gas was introduced while heating to remove hydrogen chloride in the system, thereby obtaining a yellow transparent liquid. After connecting a 3 L eggplant-shaped flask to the flask and removing phosgene in the system under reduced pressure, the contents were transferred to a 1 L four-necked flask equipped with a Dimroth condenser and a Liebig condenser by using 108 g of o-dichlorobenzene. The flask was heated while stirring at 350 rpm using an EPOCIZER (available from DIC Corporation, product name: W-100-EL) magnet stirrer, and a light boiling component was distilled off via the Dimroth condenser. Thereafter, the main distillation was distilled to obtain 321.9 g of dicyclohexylmethane-4,4'-diisocyanate (hereinafter, referred to as "H12MDI-t") having a purity of 99.3%, a cis-cis isomer: 5.5%, a cis-trans isomer: 36.4%, and a trans-trans isomer: 58.1%.

Example 7: Production of polyurethane

To 195.85 g of polytetramethylene ether glycol (available from Fuji Film Wako Pure Chemical Industries, Ltd., product name: Polytetramethylene oxide 2,000) (average molecular weight: 1,900 to 2,120), 0.2696 g of a toluene solution of dibutyltin dilaurate (available from Tokyo Kasei Co., Ltd.) (dibutyltin dilaurate: 1.0 mass%) was added, and the mixture was stirred at 80°C. Thereafter, the mixture was dried under reduced pressure at 100°C for 2 hours to distill off water and toluene. 63.31 g of H12MDI-t melted at 60°C under a nitrogen atmosphere was added, then the temperature was raised to 80°C, and the mixture was stirred under a nitrogen atmosphere for 2 hours to obtain a prepolymer. The inside of the flask was depressurized and defoamed for several minutes, and then transferred to a rotation/revolution mixer (AR-100 available from THINKY CORPORATION). Subsequently, 4.47 g of dehydrated 1,4-butanediol (available from Tokyo Chemical Industry Co., Ltd.) was added, and the resulting mixture was stirred for 2 minutes with a mixer and then defoamed for 30 seconds. The obtained reaction product was poured into a 3 mm thick mold and held at 100°C for 41 hours in a thermostat to obtain a polyurethane molded body. After curing at 23°C and 50% RH for 7 days or more for completing the reaction, it was confirmed by FT-IR measurement that the peak attributed to the isocyanate of the raw material disappeared.

Comparative Example 4: Production of polyurethane

A polyurethane molded body was obtained in the same manner as in Example 7 except that 63.30 g of commercially available dicyclohexylmethane-4,4'-diisocyanate (H12MDI, available from Tokyo Chemical Industry Co., Ltd.) was used instead of H12MDI-t.

[Method for evaluating polyurethane]

The polyurethane produced in each example was evaluated by the following method.

(1) Molecular weight (GPC measurement)

The molecular weight of the polyurethane was measured by GPC measurement. The GPC measurement was performed using N,N'-dimethylformamide to which 10 mmol/L of lithium bromide was added as an eluent. The GPC column available from Showa Denko K.K. (two Shodex GP KD-806M and one KD-802 were connected in series) and the detector Shodex RI-101 available from the same company were used to perform measurement under the conditions of a column temperature of 50°C and a solvent flow rate of 1.0 mL/min. A calibration curve was prepared using polyethylene oxide and polyethylene glycol as standard samples, and a number average molecular weight (Mn) and a weight average molecular weight (Mw) of the polyurethane were determined using this calibration curve.

(2) Durometer hardness (HDA)

The durometer hardness before and after curing was compared in accordance with "JIS K 7311: 1995 Testing methods for thermoplastic polyurethane elastomers." Two test pieces having a size of 25 mm × 12 mm × t3 mm were stacked to have a thickness of 6 mm, and the durometer hardness was measured with a durometer type A available from Teclock under a load of 1 kgf.

(3) Tensile test

The tensile strength and elongation after curing were measured in accordance with "JIS K 7311: 1995 Testing methods for thermoplastic polyurethane elastomers." The shape of the test piece was No. 3 prescribed in JIS K 6251. The polyurethane molded bodies obtained in Example 7 and Comparative Example 4 were hot-pressed at a maximum temperature of 170°C under a pressure of 10 MPa to prepare test pieces each having a thickness of 0.8 mm. Using a universal material testing machine Model 5966 available from Instron, the tensile strength was measured at a test speed of 300 mm/min, a gauge line distance of 20 mm (using a contact type Auto-X automatic extensometer), and a chuck distance of 70 mm.

(4) Crystallization temperature (DSC measurement)

A crystallization temperature of a hard segment (HS) was measured with a DSC apparatus EXSTAR DSC 7020 available from Hitachi High-Tech Science Corporation in accordance with "JIS K 7121, Testing methods for transition temperatures of plastics." An exothermic peak temperature observed when the temperature was raised from -90°C to 270°C and then lowered from 270°C to -70°C at a temperature increase/decrease rate of 10°C/min was defined as a crystallization temperature (Tc) of the hard segment.

(5) Softening temperature (DMA measurement)

The measurement was performed using EXSTAR DMS6100 available from Hitachi High-Tech Science Corporation by setting the test piece to 40 mm × 10 mm × 3 mm, under the conditions of a frequency of 1 Hz, a nitrogen atmosphere, a temperature increase rate of 4°C/min, and a measurement temperature of -120°C to 160°C. In a region where a storage elastic modulus of a rubber-like flat region was inflected, the intersection of tangents of the storage elastic moduli was determined, and this temperature was defined as the softening temperature.
The evaluation results in the polyurethane of each example are shown in Table 3

Table 3

| | Unit | Example 7 | Comparative Example 4 |
|---|---|---|---|
| Isocyanate used | - | H12MDI-t | H12MDI |

(continued)

| | | | Unit | Example 7 | Comparative Example 4 |
|---|---|---|---|---|---|
| GPC | Mn(x $10^4$) | | - | 3.73 | 3.43 |
| | Mw(x $10^4$) | | - | 14.9 | 13.3 |
| | Mw/Mn | | - | 4.00 | 3.88 |
| Durometer hardness (HDA) | Before curing | | - | 81 | 64 |
| | After curing | | - | 82 | 69 |
| Tensile test (t0.8 mm) | Tensile fracture strength | | MPa | 40.8 | 32.5 |
| | Fracture nominal strain | | % | 590 | 600 |
| | Tensile stress | At 100% elongation | MPa | 3.74 | 2.29 |
| | | At 300% elongation | MPa | 6.06 | 4.28 |
| DSC | Crystallization temperature (Tc) | | °C | 119.7 | - |
| DMA | Softening temperature | | °C | 157.9 | 60.8 |

As shown in Table 3, compared with Comparative Example 4, in Example 7, the durometer hardness after curing increased from 69 to 82, the tensile fracture strength increased from 32.5 MPa to 40.8 MPa, and thus improvement in mechanical properties was confirmed. In addition, the softening temperature increased from 60.8°C to 157.9°C in the DMA measurement, and crystallization of the hard segment was observed in the DSC measurement, and thus it was confirmed that polyurethane excellent in the mechanical properties and heat resistance was obtained by the method for producing polyurethane of the present embodiment.

Industrial Applicability

The aliphatic diamine obtained by the isomerization method of the present invention has industrial applicability as an optical material such as a plastic lens, a prism, an optical fiber, an information recording substrate, or a filter using polyamide, an epoxy curing agent, an epoxy resin, polyurethane, or the like, obtained using an aliphatic diamine.

## Claims

1. A method for isomerizing an aliphatic diamine, the method comprising isomerizing an aliphatic diamine represented by Formula (1) in the presence of:

   an imine compound obtained by dehydration condensation of an aliphatic diamine represented by Formula (1) and an aldehyde and/or a ketone; and
   at least one selected from the group consisting of an alkali metal, an alkali metal-containing compound, an alkaline earth metal, and an alkaline earth metal-containing compound,
   wherein a ratio of a total amount of the alkali metal, the alkali metal-containing compound, the alkaline earth metal, and the alkaline earth metal-containing compound used to a total amount of the aliphatic diamine used is 1.0 mol% or greater and 4.0 mol% or less,

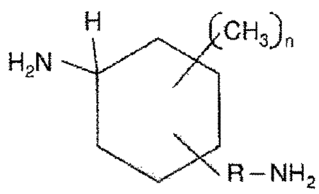

(1)

   wherein R represents a single bond or an unsubstituted aliphatic or alicyclic alkylene group having 1 to 8 carbon atoms, and n represents an integer from 0 to 5.

**2.** The method for isomerizing the aliphatic diamine according to claim 1, wherein a ratio of a total amount of the aldehyde and ketone used to the total amount of the aliphatic diamine used is 3.5 mol% or greater and 15.0 mol% or less.

**3.** The method for isomerizing the aliphatic diamine according to claim 1 or 2, wherein the ratio of the total amount of the aldehyde and ketone used to the total amount of the aliphatic diamine used is 3.5 mol% or greater and 10.0 mol% or less.

**4.** The method for isomerizing the aliphatic diamine according to any one of claims 1 to 3,

wherein the aliphatic diamine comprises an aliphatic diamine represented by Formula (1A):

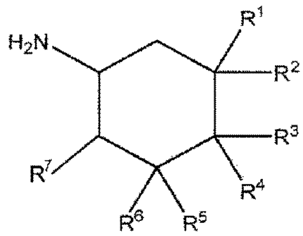

(1 A)

wherein any one of $R^1$, $R^2$, $R^3$, $R^4$, $R^5$, $R^6$, and $R^7$ is an aminoalkyl group represented by - R-$NH_2$, and the others of $R^1$, $R^2$, $R^3$, $R^4$, $R^5$, $R^6$, and $R^7$ each independently represent a hydrogen atom or a methyl group, and R represents a single bond or an unsubstituted aliphatic or alicyclic alkylene group having 1 to 8 carbon atoms.

**5.** The method for isomerizing the aliphatic diamine according to any one of claims 1 to 4, wherein the isomerization is a step of isomerizing the aliphatic diamine in the presence of the imine compound and at least one selected from the group consisting of sodium amide, metallic sodium, and sodium hydride.

**6.** The method for isomerizing the aliphatic diamine according to any one of claims 1 to 5, wherein the imine compound is an imine compound obtained by dehydration condensation of the aliphatic diamine with an aldehyde having an aromatic ring and/or a ketone having an aromatic ring.

**7.** The method for isomerizing the aliphatic diamine according to any one of claims 1 to 6, wherein the aliphatic diamine is diaminodicyclohexylmethane.

**8.** The method for isomerizing the aliphatic diamine according to any one of claims 1 to 6, wherein the aliphatic diamine is 1,3,3-trimethyl-1-(aminomethyl) aminocyclohexane.

**9.** The method for isomerizing the aliphatic diamine according to any one of claims 1 to 6, wherein the aliphatic diamine is cyclohexanediamine.

**10.** The method for isomerizing the aliphatic diamine according to any one of claims 1 to 6, wherein the aliphatic diamine is dimethyldiaminodicyclohexylmethane.

**11.** The method for isomerizing the aliphatic diamine according to any one of claims 1 to 6, wherein the aliphatic diamine is methylcyclohexanediamine.

**12.** The method for isomerizing the aliphatic diamine according to any one of claims 1 to 6,

wherein the aliphatic diamine comprises an aliphatic diamine represented by Formula (1B):

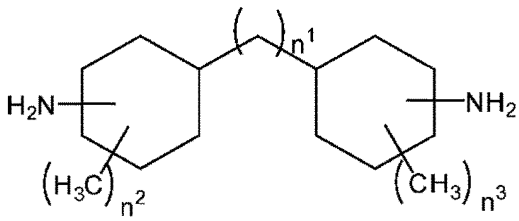

(1 B)

where $n^1$ is an integer from 0 to 2, and $n^2$ is an integer from 0 to 5, $n^3$ is an integer from 0 to 2, and a sum of $n^1$ and $n^3$ is 2 or less.

**13.** A method for producing diisocyanate, the method comprising:

isomerizing an aliphatic diamine by the method described in any one of claims 1 to 12; and
obtaining diisocyanate using the isomerized aliphatic diamine as a raw material.

**14.** A method for producing polyurethane, the method comprising:

isomerizing an aliphatic diamine by the method described in any one of claims 1 to 12;
obtaining diisocyanate using the isomerized aliphatic diamine as a raw material; and
reacting the diisocyanate obtained in the obtaining step with polyol to obtain polyurethane.

**15.** Polyurethane comprising a structure derived from the diisocyanate produced by the production method according to claim 13, wherein the production method according to claim 13 uses isomerized aliphatic diamine as a raw material, which has a trans-trans isomer proportion of 50% or greater.

**Patentansprüche**

**1.** Verfahren zum Isomerisieren eines aliphatischen Diamins, wobei das Verfahren umfasst
das Isomerisieren eines aliphatischen Diamins, dargestellt durch Formel (1), in Gegenwart von:

einer Iminverbindung, die erhalten wird durch Dehydratisierungskondensation eines aliphatischen Diamins, dargestellt durch Formel (1), und eines Aldehyds und/oder Ketons; und
mindestens einem, das ausgewählt ist aus der Gruppe bestehend aus einem Alkalimetall, einer alkalimetallhaltigen Verbindung, einem Erdalkalimetall und einer erdalkalimetallhaltigen Verbindung,
wobei das Verhältnis der Gesamtmenge des verwendeten Alkalimetalls, der alkalimetallhaltigen Verbindung, des Erdalkalimetalls und der erdalkalimetallhaltigen Verbindung zur Gesamtmenge des verwendeten aliphatischen Diamins 1,0 Mol-% oder mehr und 4,0 Mol-% oder weniger beträgt,

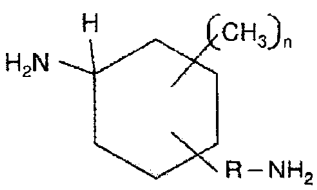

(1)

worin R eine Einfachbindung oder eine unsubstituierte aliphatische oder alicyclische Alkylengruppe mit 1 bis 8 Kohlenstoffatomen darstellt und n eine ganze Zahl von 0 bis 5 darstellt.

**2.** Verfahren zum Isomerisieren des aliphatischen Diamins gemäß Anspruch 1,
wobei das Verhältnis der Gesamtmenge des verwendeten Aldehyds und Ketons zur Gesamtmenge des verwendeten aliphatischen Diamins 3,5 Mol-% oder mehr und 15,0 Mol-% oder weniger beträgt.

**3.** Verfahren zum Isomerisieren des aliphatischen Diamins gemäß Anspruch 1 oder 2,
wobei das Verhältnis der Gesamtmenge des verwendeten Aldehyds und Ketons zur Gesamtmenge des verwendeten

aliphatischen Diamins 3,5 Mol-% oder mehr und 10,0 Mol-% oder weniger beträgt.

4. Verfahren zum Isomerisieren des aliphatischen Diamins gemäß mindestens einem der Ansprüche 1 bis 3, wobei das aliphatische Diamin ein aliphatisches Diamin, dargestellt durch die Formel (1A), umfasst:

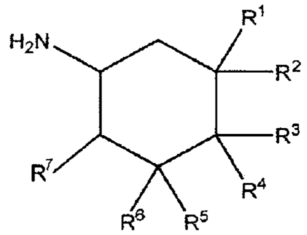

(1 A)

worin eines von $R^1$, $R^2$, $R^3$, $R^4$, $R^5$, $R^6$ und $R^7$ eine Aminoalkylgruppe, dargestellt durch $-R-NH_2$, ist und die anderen von $R^1$, $R^2$, $R^3$, $R^4$, $R^5$, $R^6$ und $R^7$ jeweils unabhängig ein Wasserstoffatom oder eine Methylgruppe darstellen, und R eine Einfachbindung oder eine unsubstituierte aliphatische oder alicyclische Alkylengruppe mit 1 bis 8 Kohlenstoffatomen darstellt.

5. Verfahren zum Isomerisieren des aliphatischen Diamins gemäß mindestens einem der Ansprüche 1 bis 4, wobei das Isomerisieren ein Schritt ist, bei dem das aliphatische Diamin in Gegenwart der Iminverbindung und mindestens einem, das ausgewählt ist aus der Gruppe bestehend aus Natriumamid, metallischem Natrium und Natriumhydrid, isomerisiert wird.

6. Verfahren zum Isomerisieren des aliphatischen Diamins gemäß mindestens einem der Ansprüche 1 bis 5, wobei die Iminverbindung eine Iminverbindung ist, die erhalten wird durch Dehydratisierungskondensation des aliphatischen Diamins mit einem Aldehyd, das einen aromatischen Ring aufweist, und/oder einem Keton, das einen aromatischen Ring aufweist.

7. Verfahren zum Isomerisieren des aliphatischen Diamins gemäß mindestens einem der Ansprüche 1 bis 6, wobei das aliphatische Diamin Diamindicyclohexylmethan ist.

8. Verfahren zum Isomerisieren des aliphatischen Diamins gemäß mindestens einem der Ansprüche 1 bis 6, wobei das aliphatische Diamin 1,3,3-Trimethyl-1-(aminomethyl)aminocyclohexan ist.

9. Verfahren zum Isomerisieren des aliphatischen Diamins gemäß mindestens einem der Ansprüche 1 bis 6, wobei das aliphatische Diamin Cyclohexandiamin ist.

10. Verfahren zum Isomerisieren des aliphatischen Diamins gemäß mindestens einem der Ansprüche 1 bis 6, wobei das aliphatische Diamin Dimethyldiamindicyclohexylmethan ist.

11. Verfahren zum Isomerisieren des aliphatischen Diamins gemäß mindestens einem der Ansprüche 1 bis 6, wobei das aliphatische Diamin Methylcyclohexandiamin ist.

12. Verfahren zum Isomerisieren des aliphatischen Diamins gemäß mindestens einem der Ansprüche 1 bis 6, wobei das aliphatische Diamin ein aliphatisches Diamin, dargestellt durch die Formel (1B), umfasst:

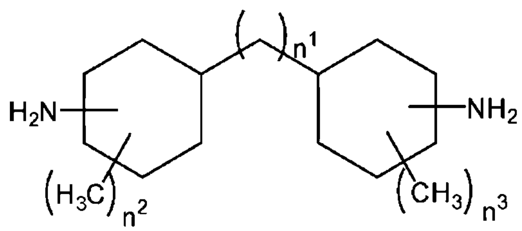

(1 B)

worin $n^1$ eine ganze Zahl von 0 bis 2 ist und $n^2$ eine ganze Zahl von 0 bis 5 ist, $n^3$ eine ganze Zahl von 0 bis 2 ist und die

Summe von $n^1$ und $n^3$ 2 oder weniger beträgt.

**13.** Verfahren zur Herstellung von Diisocyanat, wobei das Verfahren umfasst:

Isomerisieren eines aliphatischen Diamins durch das in mindestens einem der Ansprüche 1 bis 12 beschriebene Verfahren; und
Gewinnen von Diisocyanat unter Verwendung des isomerisierten aliphatischen Diamins als Rohmaterial.

**14.** Verfahren zur Herstellung von Polyurethan, wobei das Verfahren umfasst:

Isomerisieren eines aliphatischen Diamins durch das in mindestens einem der Ansprüche 1 bis 12 beschriebene Verfahren;
Gewinnen von Diisocyanat unter Verwendung des isomerisierten aliphatischen Diamins als Rohmaterial; und
Umsetzen des in dem Gewinnungsschritt gewonnenen Diisocyanats mit Polyol, um Polyurethan zu gewinnen.

**15.** Polyurethan, umfassend eine Struktur, die von dem Diisocyanat abgeleitet ist, das durch das Herstellungsverfahren gemäß Anspruch 13 hergestellt wurde, wobei das Herstellungsverfahren gemäß Anspruch 13 isomerisiertes aliphatisches Diamin als Rohmaterial verwendet, das einen Trans-Trans-Isomeranteil von 50 % oder mehr aufweist.

**Revendications**

**1.** Procédé d'isomérisation d'une diamine aliphatique, le procédé comprenant
l'isomérisation d'une diamine aliphatique représentée par la Formule (1) en présence de :

un composé d'imine obtenu par condensation par déshydratation d'une diamine aliphatique représentée par la Formule (1) et d'un aldéhyde et/ou d'une cétone ; et
au moins un élément choisi dans le groupe consistant en un métal alcalin, un composé contenant un métal alcalin, un métal alcalino-terreux, et un composé contenant un métal alcalino-terreux,
dans lequel un rapport entre une quantité totale du métal alcalin, du composé contenant un métal alcalin, du métal alcalino-terreux et du composé contenant un métal alcalino-terreux utilisée et une quantité totale de la diamine aliphatique utilisée est supérieur ou égal à 1,0 % en moles et inférieur ou égal à 4,0 % en moles,

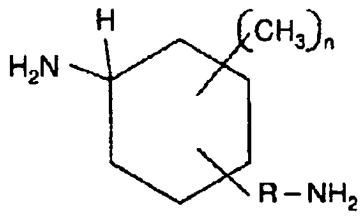

(1)

dans lequel R représente une liaison simple ou un groupe alkylène aliphatique ou alicyclique non substitué présentant de 1 à 8 atomes de carbone, et n représente un nombre entier de 0 à 5.

**2.** Procédé d'isomérisation de la diamine aliphatique selon la revendication 1,
dans lequel un rapport entre une quantité totale de l'aldéhyde et de la cétone utilisée et la quantité totale de la diamine aliphatique utilisée est supérieur ou égal à 3,5 % en moles et inférieur ou égal à 15,0 % en moles.

**3.** Procédé d'isomérisation de la diamine aliphatique selon la revendication 1 ou 2,
dans lequel le rapport entre la quantité totale de l'aldéhyde et de la cétone utilisée et la quantité totale de la diamine aliphatique utilisée est supérieur ou égal à 3,5 % en moles et inférieur ou égal à 10,0 % en moles.

**4.** Procédé d'isomérisation de la diamine aliphatique selon l'une quelconque des revendications 1 à 3,

dans lequel la diamine aliphatique comprend une diamine aliphatique représentée par la Formule (1A) :

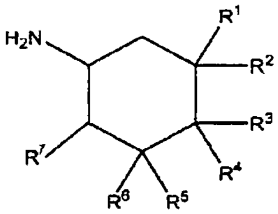

(1 A)

dans lequel l'un quelconque de $R^1$, $R^2$, $R^3$, $R^4$, $R^5$, $R^6$ et $R^7$ est un groupe aminoalkyle représenté par $-R-NH_2$, et les autres de $R^1$, $R^2$, $R^3$, $R^4$, $R^5$, $R^6$ et $R^7$ représentent chacun indépendamment un atome d'hydrogène ou un groupe méthyle, et R représente une liaison simple ou un groupe alkylène aliphatique ou alicyclique non substitué présentant de 1 à 8 atomes de carbone.

**5.** Procédé d'isomérisation de la diamine aliphatique selon l'une quelconque des revendications 1 à 4, dans lequel l'isomérisation est une étape d'isomérisation de la diamine aliphatique en présence du composé d'imine et d'au moins un élément choisi dans le groupe consistant en l'amide de sodium, le sodium métallique et l'hydrure de sodium.

**6.** Procédé d'isomérisation de la diamine aliphatique selon l'une quelconque des revendications 1 à 5, dans lequel le composé d'imine est un composé d'imine obtenu par condensation par déshydratation de la diamine aliphatique avec un aldéhyde présentant un cycle aromatique et/ou une cétone présentant un cycle aromatique.

**7.** Procédé d'isomérisation de la diamine aliphatique selon l'une quelconque des revendications 1 à 6, dans lequel la diamine aliphatique est le diaminodicyclohexylméthane.

**8.** Procédé d'isomérisation de la diamine aliphatique selon l'une quelconque des revendications 1 à 6, dans lequel la diamine aliphatique est le 1,3,3-triméthyl-1-(aminométhyl) aminocyclohexane.

**9.** Procédé d'isomérisation de la diamine aliphatique selon l'une quelconque des revendications 1 à 6, dans lequel la diamine aliphatique est la cyclohexanediamine.

**10.** Procédé d'isomérisation de la diamine aliphatique selon l'une quelconque des revendications 1 à 6, dans lequel la diamine aliphatique est le diméthyldiaminodicyclohexylméthane.

**11.** Procédé d'isomérisation de la diamine aliphatique selon l'une quelconque des revendications 1 à 6, dans lequel la diamine aliphatique est la méthylcyclohexanediamine.

**12.** Procédé d'isomérisation de la diamine aliphatique selon l'une quelconque des revendications 1 à 6,

dans lequel la diamine aliphatique comprend une diamine aliphatique représentée par la Formule (1B) :

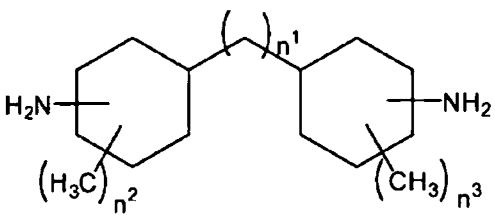

(1 B)

où $n^1$ est un nombre entier compris entre 0 et 2, et $n^2$ est un nombre entier compris entre 0 et 5, $n^3$ est un nombre entier compris entre 0 et 2, et une somme de $n^1$ et de $n^3$ est inférieure ou égale à 2.

**13.** Procédé de production de diisocyanate, le procédé comprenant :

l'isomérisation d'une diamine aliphatique par le procédé décrit dans l'une quelconque des revendications 1 à 12 ; et
l'obtention de diisocyanate en utilisant la diamine aliphatique isomérisée comme matière première.

**14.** Procédé de production de polyuréthane, le procédé comprenant :

l'isomérisation d'une diamine aliphatique par le procédé décrit dans l'une quelconque des revendications 1 à 12 ;
l'obtention de diisocyanate en utilisant la diamine aliphatique isomérisée comme matière première ; et
la mise en réaction du diisocyanate obtenu lors de l'étape d'obtention avec du polyol pour obtenir du polyuréthane.

**15.** Polyuréthane comprenant une structure dérivée du diisocyanate produit par le procédé de production selon la revendication 13, dans lequel le procédé de production selon la revendication 13 utilise comme matière première de la diamine aliphatique isomérisée dont la proportion d'isomères trans-trans est égale ou supérieure à 50 %.

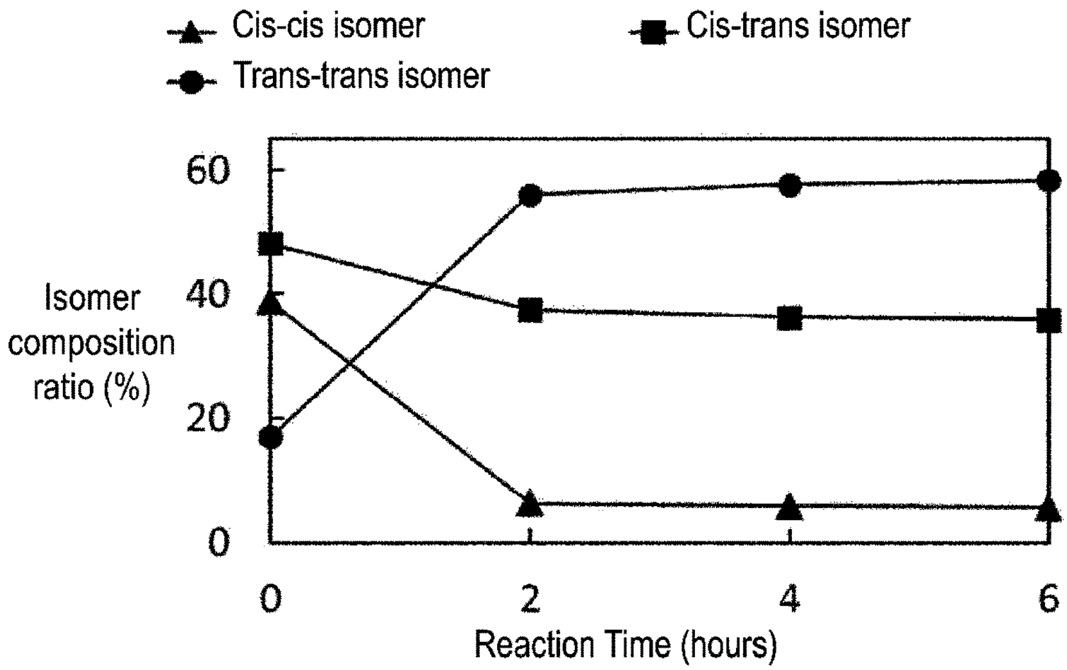
Cis-cis isomer
Cis-trans isomer
Trans-trans isomer
Isomer composition ratio (%)
60
40
20
0
0
2
4
6
Reaction Time (hours)

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 7188128 A
- DE 4211454
- WO 2016143536 A
- WO 2016143537 A
- WO 2016143538 A
- WO 2009051114 A
- CN 102627569